# EUROPEAN PATENT SPECIFICATION

(11) **EP 2 635 110 B1**
(45) Date of publication and mention of the grant of the patent: **09.05.2018**
(21) Application number: 11838895.8
(22) Date of filing: 04.11.2011
(51) Int. Cl.: G01N 33/50, A01K 67/00

(54) **CONTROL AND CHARACTERIZATION OF PSYCHOTIC STATES**
STEUERUNG UND CHARAKTERISIERUNG PSYCHOTISCHER ZUSTÄNDE
CONTRÔLE ET CARACTÉRISATION D'ÉTATS PSYCHOTIQUES

(30) Priority: 05.11.2010 US 410720 P; 05.11.2010 US 410725 P
(43) Date of publication of application: 11.09.2013
(73) Proprietor: The Board of Trustees of the Leland Stanford Junior University, Stanford CA 94305-2038 (US)
(72) Inventor: DEISSEROTH, Karl, Palo Alto, CA 94305 (US); SOHAL, Vikaas, Sam Francisco, CA 94127 (US); GUNAYDIN, Lisa, Stanford, CA 94305 (US)
(74) Representative: Brasnett, Adrian Hugh
(86) International application number: PCT/US2011/059383
(87) International publication number: WO 2012/061741

(56) References cited:
- WO-A2-2010/056970
- WO-A2-2010/056970
- WO-A2-2011/005978
- WO-A2-2012/061741
- WO-A2-2012/061744
- US-A1- 2008 060 088
- US-A1- 2009 088 680
- ARENKIEL B.R. ET AL.: "In vivo light-induced activation of neuralcircuitry in transgenic mice expressing Channelrhodopsin-2", NEURON, vol. 54, 2007, pages 205-218, XP002718529,
- WANG H ET AL: "High-speed mapping of synaptic connectivity using photostimulation in Channel rhodopsin-2 transgenic mice", PROCEEDINGS OF THE NATIONAL ACADEMY OF SCIENCES, NATIONAL ACADEMY OF SCIENCES, US, vol. 104, no. 19, 8 May 2007 (2007-05-08), pages 8143-8148, XP009159082, ISSN: 0027-8424, DOI: 10.1073/PNAS.0700384104
- MICHELE S MILELLA ET AL: "Opposite roles of dopamine and orexin in quinpirole-induced excessive drinking: a rat model of psychotic polydipsia", PSYCHOPHARMACOLOGY, SPRINGER, BERLIN, DE, vol. 211, no. 3, 16 June 2010 (2010-06-16) , pages 355-366, XP019838998, ISSN: 1432-2072
- YIZHAR O. ET AL.: "Neocortical excitation/inhibition balance in information processing and social dysfunction.", NATURE, vol. 477, 8 September 2011 (2011-09-08), pages 171-178, XP002718530,
- WANG H. ET AL.: 'High-speed mapping of synaptic connectivity using photostimulation in Channelrhodopsin-2 transgenic mice.' PROC NATL ACAD SCI USA vol. 104, no. 19, 08 May 2007, pages 8143 - 8148, XP009159082
- SIMMONS M.A. ET AL.: 'Localization and function of NK(3) subtype tachykinin receptors of layer V pyramidal neurons of the guinea-pig medial prefrontal cortex.' NEUROSCIENCE vol. 156, no. 4, 28 October 2008, pages 987 - 994, XP025586169
- ERIC E NELSON ET AL: "Non-Human Primates: Model Animals for Developmental Psychopathology", NEUROPSYCHOPHARMACOLOGY., vol. 34, no. 1, 17 September 2008 (2008-09-17), pages 90-105, XP055372477, US ISSN: 0893-133X, DOI: 10.1038/npp.2008.150
- FENG ZHANG ET AL: "The Microbial Opsin Family of Optogenetic Tools", CELL, vol. 147, no. 7, December 2011 (2011-12), pages 1446-1457, XP028348924, ISSN: 0092-8674, DOI: 10.1016/J.CELL.2011.12.004 [retrieved on 2011-12-08]
- LIEF FENNO ET AL: "The Development and Application of Optogenetics", ANNUAL REVIEW OF NEUROSCIENCE, vol. 34, no. 1, 21 July 2011 (2011-07-21), pages 389-412, XP055135085, ISSN: 0147-006X, DOI: 10.1146/annurev-neuro-061010-113817
- CA JONES ET AL: "Animal models of schizophrenia", BRITISH JOURNAL OF PHARMACOLOGY, vol. 164, no. 4, 1 October 2011 (2011-10-01), pages 1162-1194, XP055282219, BASINGSTOKE, HANTS; GB ISSN: 0007-1188, DOI: 10.1111/j.1476-5381.2011.01386.x

## Description

### FIELD OF THE INVENTION

This application pertains to methods for inducing psychosis in non-human animals using light-responsive opsin proteins expressed on the plasma membranes of a subset of layer V pyramidal neurons in the prefrontal cortex and methods for identifying or screening a compound that may be used for treating psychosis.

### BACKGROUND OF THE INVENTION

Schizophrenia affects approximately 1% of the population worldwide and ranks among the top 10 causes of disability in developed countries, but current pharmacotherapies are often ineffective and induce serious treatment-limiting side effects. It is widely believed that dysfunction of the prefrontal cortex (PFC) underlies many of the most debilitating aspects of schizophrenia (1, 2); however, it has not been possible to causally link specific aspects of cellular physiology to prefrontal dysfunction in schizophrenia. To search for possible cellular underpinnings of the psychotic behavior and impaired cognition observed in schizophrenia and related conditions, we sought to identify patterns of cellular behavior that (1) occur in neurons relevant to psychotic behaviors, (2) result from multiple pharmacologic or genetic manipulations linked to schizophrenia, and (3) hold face validity as cellular endophenotypes for psychosis.

Many debilitating aspects of schizophrenia are thought to result from dysfunction of the prefrontal cortex, but the physiology of this dysfunction is mysterious, as specific pathogenic patterns of activity in prefrontal neurons remain unknown. Identifying and understanding the neural pathways linked to psychosis-related patterns of activity within the PFC region may aid in the discovery of pharmacological therapies to treat patients with schizophrenia. However, there remains a need for a useful animal model system for schizophrenia that would allow for identification of these intricate neural pathogenic pathways. Such an animal model system would allow for screening and identification of pharmacological therapies that improve the pathogenic patterns of neural activity that contribute to the symptoms of schizophrenia.

Arenkiel et al, Neuron 54, 205-218, April 19, 2007, describe *in vivo* light-induced activation of neural circuitry in transgenic mice expressing channelrhodopsin-2. Wang et al, Proc Natl Acad Sci USA 104, 8143-8148, 2007, describe high-speed mapping of synaptic connectivity using photostimulation in channelrhodopsin-2 transgenic mice. Miella et al, Psychophannacology (2010) 211:355- 366, describe opposite roles of dopalnine and orexin in quinpirole-induced excessive drinking in a rat model of psychotic polydipsia. US 2009/0088680 relates to an optical tissue interface method and an apparatus for stimulating cells. US 2008/0060088 relates to phospholipase C betal knockout mice as a model system for testing schizophrenia drugs. WO 2010/056970 describes optical stimulation of target cells using light, e.g., *in vivo* or *in vitro.* Yizhar et al, Nature 477, 171-178, 2011, describes neocortical excitation/inhibition balance in information processing and social dysfunction. WO 2012/061744 describes step function opsins and methods for their use.

### SUMMARY OF THE INVENTION

The present invention provides a method of inducing psychosis in a non-human mammal comprising a light-responsive opsin expressed on the cell membrane of a subset of layer V pyramidal neurons in the prefrontal cortex, wherein the light-responsive opsin is Chlamydomonas reinhardtii ChR2 or a variant thereof having at least 90% amino acid sequence identity to SEQ ID NO:1, the method comprising activating the light-responsive opsin by light, wherein the light activation of the opsin induces depolarization of the cell membrane, thereby inducing a psychotic state in the mammal.

The invention also provides a method of screening a compound that may be useful for treating psychosis, the method comprising: measuring a psychotic state of a non-human mammal before and after administering the compound to the prefrontal cortex of the mammal, wherein the non-human mammal comprises a light-responsive opsin expressed on the cell membrane of a subset of layer V pyramidal neurons in the prefrontal cortex, wherein the light-responsive opsin is *Chlamydomonas reinhardtii* ChR2 or a variant thereof having at least 90% amino acid sequence identity to SEQ ID NO:1, and wherein the psychotic state is induced by light activation of the light-responsive opsin, wherein light activation of the opsin induces depolarization of the membrane; wherein an improvement in one or more psychotic state measurements after the administration of the compound indicates that the compound may be useful for treating psychosis.

The invention further provides a method of screening a compound that may be useful for treating psychosis, comprising: measuring a cellular response in a prefrontal cortex tissue slice, obtained from a non-human mammal comprising a light-responsive opsin expressed on the cell membrane of a subset of layer V pyramidal neurons in the prefrontal cortex before and after incubating the tissue slice with the compound, wherein the light-responsive opsin is *Chlamydomonas reinhardtii* ChR2 or a variant thereof having at least 90% amino acid sequence identity to SEQ ID NO:1, wherein the cellular response is induced by the membrane depolarization of the neurons induced by activation of the light-responsive opsin; wherein an improvement in a cellular response readouts after incubation with the compound indicates that the compound may be useful for treating psychosis.

The present disclosure also sets out ("provides") a number of other products and methods as discussed herein below which may facilitate understanding of the present invention, which is defined by the claims.

In some aspects, provided herein are non-human animals comprising a light-responsive opsin expressed on the cell membrane of a subset of layer V pyramidal neurons in the prefrontal cortex, wherein light activation of the opsin induces depolarization of the membrane, and wherein the illumination of the opsin with the light induces psychosis of the animal. In some embodiments, the subset of layer V pyramidal neurons have a single large apical dendrite. In some embodiments, the opsin is selected from the group consisting of ChR2, VChR1, and DChR. In another embodiment, the opsin is selected from the group consisting of SFO, SSFO, C1V1, C1V1-E122T, C1V1-E162T, and C1V1-E122T/E162T.

In other aspects, provided herein are methods of inducing psychosis in a non-human animal comprising expressing a light-responsive opsin on the cell membrane of a subset of layer V pyramidal neurons in the prefrontal cortex in the animal, wherein the opsin induces depolarization of the membrane by light, and wherein illumination of the opsin with the light induces psychosis of the animal. In other aspects, provided herein are methods of inducing psychosis in a non-human animal, comprising activating a light-responsive opsin by light, wherein the light-responsive opsin is expressed on the cell membrane of a subset of layer V pyramidal neurons in the prefrontal cortex in the animal, and wherein the light activation of the opsin induces depolarization of the cell membrane and induces psychosis in the animal. In some embodiments, the subset of layer V pyramidal neurons have a single large apical dendrite. In some embodiments, the opsin is selected from the group consisting of ChR2, VChR1, and DChR. In another embodiment, the opsin is selected from the group consisting of SFO, SSFO, C1V1, C1V1-E122T, C1V1-E162T, and C1V1-E122T/E162T.

In other aspects, provided herein are prefrontal cortex tissue slices comprising a subset of layer V pyramidal neurons, wherein a light-responsive opsin is expressed on the cell membrane of the apical dentrites in layer V pyramidal neurons, and light activation of the light-responsive opsin induces depolarization of the membrane. In some embodiments, the subset of layer V pyramidal neurons have a single large apical dendrite. In some embodiments, the opsin is selected from the group consisting of ChR2, VChR1, and DChR. In another embodiment, the opsin is selected from the group consisting of SFO, SSFO, C1V1, C1V1-E122T, C1V1-E162T, and C1V1-E122T/E162T.

In still other aspects, provided herein are methods of screening a compound that may be useful for treating psychosis, comprising measuring psychotic state of a non-human animal before and after administering the compound to the prefrontal cortex of the animal, wherein the psychotic state is induced by light activation of a light-responsive opsin expressed on the cell membrane of a subject of layer V pyramidal neurons in the animal, and activation of the opsin induces depolarization of the membrane; wherein an improvement in one or more of a psychotic state measurements after the administration of the compound indicates that the compound may be useful for treating psychosis In some embodiments, the psychotic state measurement is a behavioral measurement. In some embodiments, the psychotic state measurement is a cellular measurement. In some embodiments, the method further comprises a step of administering a D2 agonist to the animal before administration of the compound.

In some aspects, provided herein are methods of screening a compound that may be useful for treating psychosis, comprising: measuring a psychotic state of a prefrontal cortex tissue slice before and after incubating the tissue slice with the compound, wherein the prefrontal cortex tissue slice comprises a subject of layer V pyramidal neurons and a light-responsive opsin is expressed on the cell membrane of the subject of layer V pyramidal neurons, wherein the psychotic state is induced by the membrane depolarization of the neurons induced by activation of the light-responsive opsin; wherein an improvement in one or more of a psychotic state readouts after incubation with the compound indicates that the compound may be useful for treating psychosis. In some embodiments, the psychotic state measurement is a cellular measurement. In some embodiments, the method further comprises a step of incubating a D2 agonist with the prefrontal cortex tissue slice before incubation with the compound.

The present disclosure relates to identifying neural cell populations involved in various psychiatric disorders, as described herein. While the present disclosure is not necessarily limited in these contexts, various aspects of the invention may be appreciated through a discussion of examples using these and other contexts.

Aspects of the present disclosure are directed to a method of identifying neural cell populations implicated in various psychiatric disorders. The method includes providing optical stimulation to a target neuron population that expresses a light-responsive opsin. A first electrical pattern of a target neuron cell population in response to the optical stimulation is measured. Then, a drug, known to induce a disorder of interest, is introduced to the target neuron cell population. Optical stimulation is again provided to the target neuron population. A subsequent electrical pattern of the target neuron cell population, in response to the optical stimulation, is measured. The first electrical pattern and the subsequent electrical pattern are then compared. The comparison of the electrical patterns can be used to determine, for example, which neurons are involved in creating a disease-like state. After a specific neuron population has been identified, subsequent potential treatments can be targeted at the specific neuron population. Alternatively, additional studies may be done on the specific neuron population to determine the mechanisms behind the aberrant behavior, for example.

Aspects of the present disclosure are directed to comparing the electrical activity of a target neuron population of interest before and after the introduction of a drug known to induce a disorder of interest. A stimulus is provided both before and after the introduction of the drug, and the electrical response patterns to the drug are compared. The stimulus can be, for example, an optical stimulus, an electrical stimulus, or magnetic stimulus.

In certain specific embodiments, an area of interest within the brain is chosen. This choice can be made based on previous knowledge regarding the function of the brain and the mechanisms by which drugs, targeted at psychiatric disorders, work. For example, Layer V pyramidal neurons were previously linked to certain forms of psychosis. Aspects of the present disclosure allowed for the identification of a subset of pyramidal neurons linked to psychotic behavior, by examining the layer V pyramidal neurons using a combination of optical stimulation and induction of aberrant behavior in a subject by introducing substances previously found to induce the aberrant behavior.

This identification of a neuron population linked to psychotic behavior allows for the efficient testing of possible treatments for various psychotic behaviors. Once the reaction of particular neuron during psychosis is known and compared to the reaction when psychosis has not been introduced, various treatments can be tested based on the treatment's ability to return the neuron reaction to its baseline state.

Certain aspects of the present disclosure are also directed to gaining a better understanding of the mechanisms within the brain and a particular neuron population that cause psychosis. After a particular neuron of interest is identified, the channels within the neuron, as well as the pathways connecting the neuron to other neurons, can be studied in greater detail. This can lead to new insights regarding the cause of various forms of psychosis.

The present disclosure further relates to specific neural cell populations involved in various psychiatric disorders including psychosis (and/or symptoms of psychosis), as described herein. While the present disclosure is not necessarily limited in these contexts, various aspects of the invention may be appreciated through a discussion of examples using these and other contexts.

Aspects of the present disclosure are directed to a method of involving a specific neural cell populations implicated in various psychiatric disorders, including psychosis (and/or the symptoms of psychosis). The method includes providing optical stimulation to a target neuron population that expresses a light responsive opsin. A first electrical pattern of a target neuron cell population in response to the optical stimulation is measured. Then, a drug, known to induce symptoms of psychosis, is introduced to the target neuron cell population. Optical stimulation is again provided to the target neuron population. A subsequent electrical pattern of the target neuron cell population, in response to the optical stimulation, is measured. The first electrical pattern and the subsequent electrical pattern are then compared. The comparison of the electrical patterns can be used to determine, for example, which neurons are involved in creating a disease-like state. After a specific neuron population has been identified, subsequent potential treatments can be targeted at the specific neuron population. Alternatively, additional studies may be done on the specific neuron population to determine the mechanisms behind the aberrant behavior, for example. In certain embodiments, the additional studies can be performed using a variety of stimuli including optical stimuli, electrical stimuli, and/or magnetic stimuli.

Aspects of the present disclosure are directed to inducing a disease state by controlling properties of a target neuron population known to be involved in psychosis. The neurons of the target neuron population have a single, large apical dendrite. The target neuron population is modified with a light-responsive molecule. Light is provided to the target neuron population, thereby activating the light-responsive molecule. A drug is introduced to the target neuron population causing the membrane potential of the neurons to remain elevated after removal of the light. The elevated membrane potential results in a modified cell response to stimulus, as well as activation of the neuron when no stimulus is present. Experimental results show that a subject, who has this neuron activity induced in certain neuron populations having a single, large apical dendrite, exhibits behaviors consistent with psychosis.

In more specific embodiments, this modified neuron activity is used as an aid in determining possible treatments for psychosis. The disease state can be induced before various potential treatments are tested. The testing can include introducing the treatment to the neurons, stimulating the neurons, and comparing the response in the presence of the treatment to the response in the absence of the treatment.

Various embodiments, relating to and/or using such methodology and apparatuses, can be appreciated by the skilled artisan, particularly in view of the figures and/or the following discussion. The above overview is not intended to describe each illustrated embodiment or every implementation of the present disclosure. For information regarding details of other embodiments, experiments and applications that can be combined in varying degrees with the teachings herein, reference may be made to the teachings and underlying references provided in the Examples which form a part of this patent document.

### BRIEF DESCRIPTION OF THE DRAWING

Various example embodiments may be more completely understood in consideration of the following detailed description in connection with the accompanying drawings, in which:

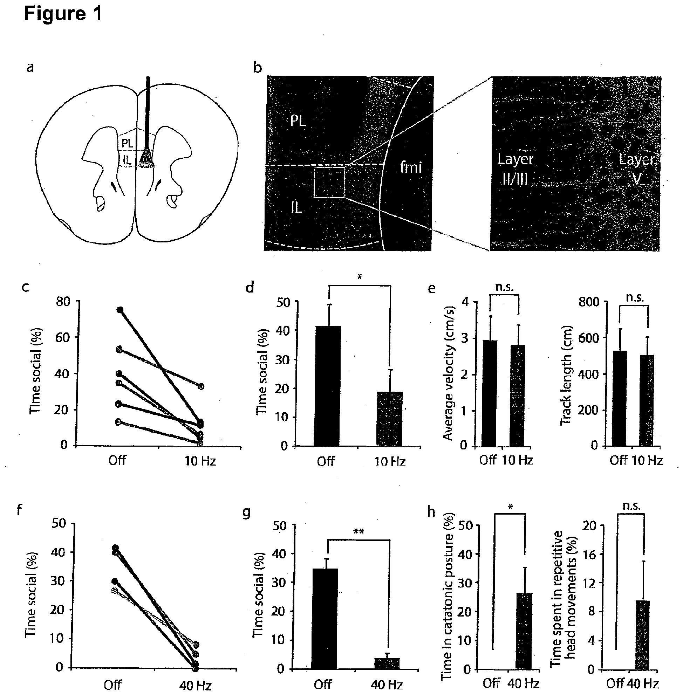

**FIG. 1** demonstrates psychotic-like behaviors induced by optical stimulation of infralimbic layer V pyramidal neurons in Thy1::ChR2 transgenic mice. **A)** Schematic representation of unilateral optical fiber placement above infralimbic cortex. **B)** Confocal images of coronal slices of prefrontal cortex in Thy1::ChR2-EYFP mice, showing optical fiber placement above layer V infralimbic cortex (left panel) and ChR2-EYFP expression in layer V neurons (right panel). C) Low-frequency optical stimulation of layer V neurons with 473 nm blue light (10 Hz, 5-ms pulse width) significantly decreased social exploration of a novel juvenile in 6 of 6 Thy1::ChR2-EYFP animals tested (p = 0.03; light on/ light off epochs interleaved). **D)** Summary data for effects of 10 Hz stimulation on social exploration as shown in **(A). E)** Summary open field data showing no effect of 10 Hz optical stimulation on overall velocity (left) or track length (right) of Thy1::ChR2 animals. **F)** Gamma-band optical stimulation of layer V neurons with 473 nm blue light (40 Hz, 5-ms pulse width) even more powerfully eliminated social exploration of a novel juvenile in 6 of 6 animals tested (p < 0.01; light on/ light off epochs interleaved). **G)** Summary data for effects of 40 Hz stimulation on social exploration as shown in **(F). H)** 40 Hz optical stimulation significantly increased time spent in a catatonic-like rigid posture in 3 of 6 mice tested (p < 0.05), and a trend was observed toward increased time spent engaging in repetitive side-to-side head movements in 2 of 6 mice tested (p = 0.13).

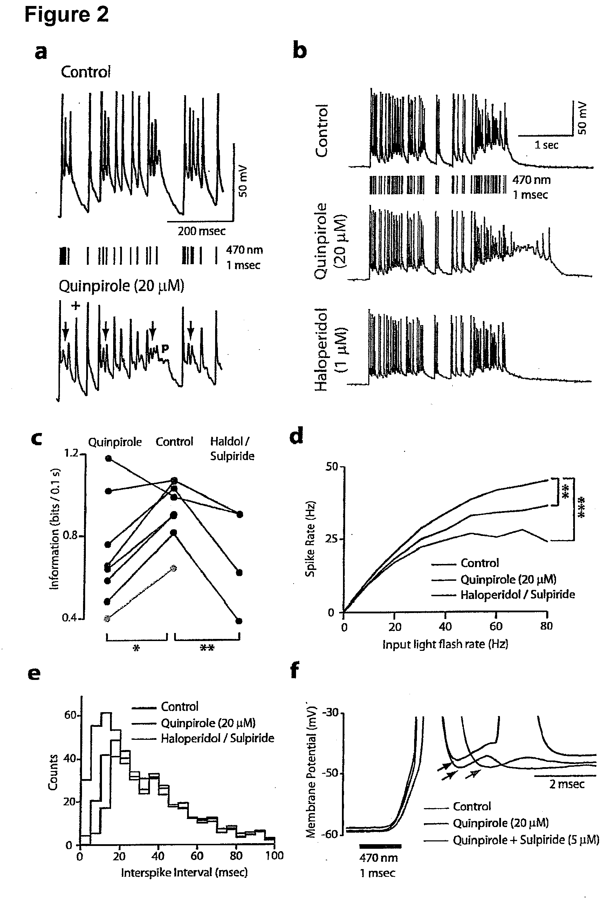

**FIG. 2** shows that the D2 agonist quinpirole modulates responses of prefrontal networks *in vitro* to ChR2 stimulation in Thy1::ChR2 transgenic mice. **A)** Responses of a layer V pyramidal neuron to a trains of light flashes (470 nm, 1 msec) in control conditions (top, black trace), and in quinpirole (20 µM; purple, bottom trace). After applying quinpirole, some light flashes which previously evoked spikes no longer do so (arrows), some new spikes, unrelated to light flashes, occur ("+"), and plateau potentials are observed ("p"). **B)** After applying quinpirole (20 µM; purple, middle trace), this layer V pyramidal neuron exhibits a prolonged depolarization that outlasts the period of light stimulation and produces spiking. The prolonged depolarization is abolished after washing out quinpirole and applying haloperidol (1 µM; green, bottom trace). **C)** The amount of information the spike rate transmits about the rate of light flashes in layer V pyramidal neurons in which quinpirole elicits an activity-dependent depolarization (n = 8 cells in control and 20 µM quinpirole; n = 4 cells in haloperidol 0.2-2 µM or sulpiride 5 µM). **D)** The rate of spikes as a function of the rate of light flashes, for layer V pyramidal neurons in which quinpirole elicits an activity-dependent depolarization (as illustrated in **B)** (n = 4 cells in each condition; haloperidol 0.2-2 µM; sulpiride 5 µM). The control (top trace ending on the right portion of **(D)),** quinpirole (middle trace ending on the right portion of **(D)),** and haloperidol/sulpiride trace (bottom trace ending on the right portion of **(D))** are shown in **(D). E)** The number of spikes as a function of interspike interval for the same cells depicted in **C.** The control (top trace beginning from on the left portion of **(E)),** quinpirole (middle trace beginning from the left portion of **(E)),** and haloperidol/sulpiride trace (bottom trace beginning from the right portion **of (E))** are shown in **(E). F)** responses of a layer V pyramidal neuron (in which quinpirole elicits an activity-dependent depolarization) to a combination of ChR2 and network-driven activity after a single 1 msec light flash in Control conditions (black trace; top trace ending on the right portion of **(F)),** quinpirole (20 µM; purple trace; bottom trace ending on the right portion of **(F)),** and quinpirole + sulpiride (5 µM; green trace; middle trace ending on the right portion of **(F)).** Arrows indicate the spike AHP.

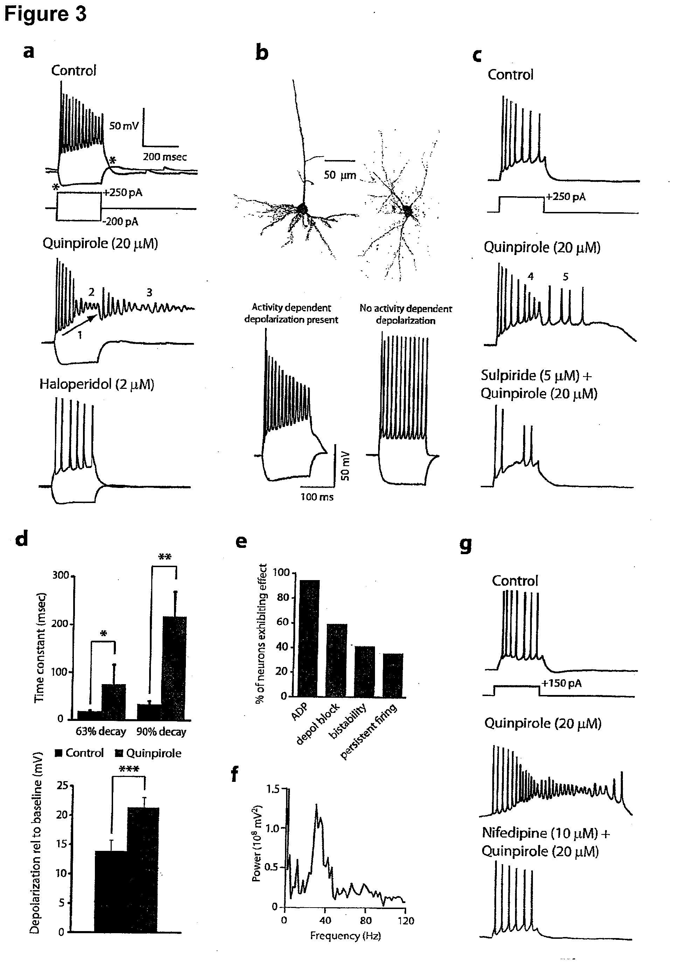

**FIG. 3** demonstrates that D2 receptor activation elicits an activity-dependent depolarization mediated by L-type Ca²⁺ channels. **A, C, G)** Responses of layer V pyramidal neurons to hyperpolarizing and/or depolarizing current pulses in various pharmacologic conditions. **B)** Morphology of layer V pyramidal neurons that do (left) and do not (right) exhibit the activity-dependent depolarization and afterdepolarization during responses to depolarizing current pulses in quinpirole (responses to depolarizing and hyperpolarizing current pulses below each cell). **D)** Top: Time constants for the membrane potential to decay by 63% or 90% towards baseline after a 250 pA depolarizing current pulse in control conditions (black; left bar) or quinpirole (purple; right bar). Note that we have excluded 3 cells that become bistable in quinpirole (i.e. the membrane potential fails to return to baseline for > 1 second). Bottom: The membrane potential (relative to baseline) 10 msec after the end of a 250 pA depolarizing current pulse in control conditions (black) or quinpirole (purple). **E)** Fraction of layer V pyramidal neurons with a prominent sag and rebound afterdepolarization that exhibited an afterdepolarization (ADP) following depolarizing current injection, depolarization blockade of spiking (depol block), bistability, or persistent firing that outlasted the period of depolarizing current injection, after application of 20 µM quinpirole. **F)** Power spectrum of the persistent activity observed in quinpirole following a depolarizing current pulse (calculated from the trace in panel **A).**

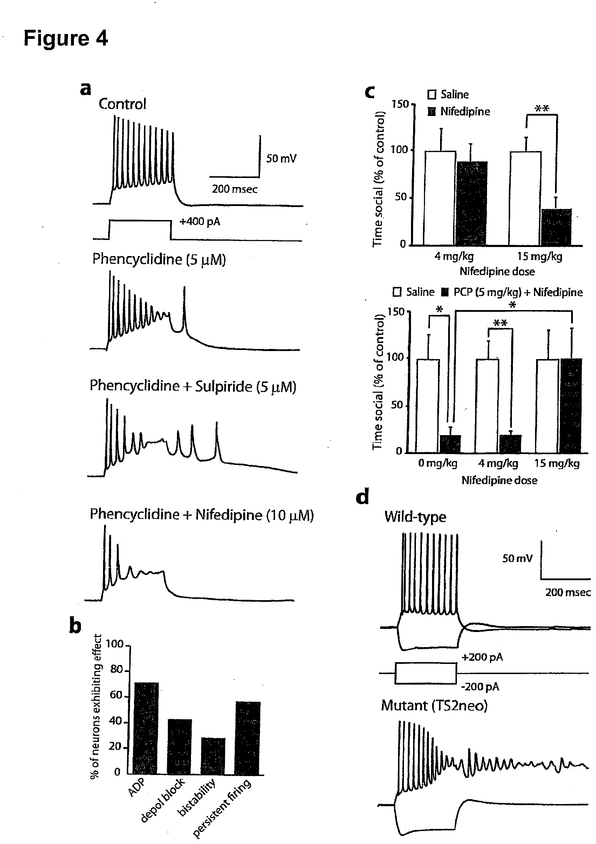

**FIG. 4** shows that phencyclidine (PCP) also elicits an activity-dependent depolarization via L-type Ca²⁺ channels. **A)** Responses of a layer V pyramidal neuron to depolarizing current pulses. After applying PCP (5 µM; middle two traces), the neuron exhibits an afterdepolarization and persistent firing that outlast the period of current injection. These are not reversed by the D2 antagonist sulphide (5 µM; green trace), but are blocked by the L-type Ca²⁺ channel antagonist nifedipine (10 µM; gray trace). **B)** Fraction of layer V pyramidal neurons with a prominent sag and rebound afterdepolarization that exhibited an afterdepolarization (ADP) following depolarizing current injection, depolarization blockade of spiking (depol block), bistability, or persistent firing that outlasted the period of depolarizing current injection, after application of 5 µM PCP. **C)** Top: Nifidepine impairs social exploration in a dose-dependent fashion (n = 8 mice in each group). Bottom: PCP impairs social exploration, but nifedipine ameliorates this deficit in PCP-treated mice in a dose-dependent fashion (n = 8 mice in each group). **D)** Responses of layer V pyramidal neurons to hyperpolarizing and depolarizing current pulses in a wild-type mouse, and in the TS2neo knock-in mouse designed as a CACNA1C gain of function gene (20). * = p < 0.05, ** = p < 0.01. The effect was present in 4/4 mutant cells and 0/5 wild-type cells with a large sag and rebound depolarization in response to hyperpolarizing current injection (p < 0.01 by Fisher's exact test).

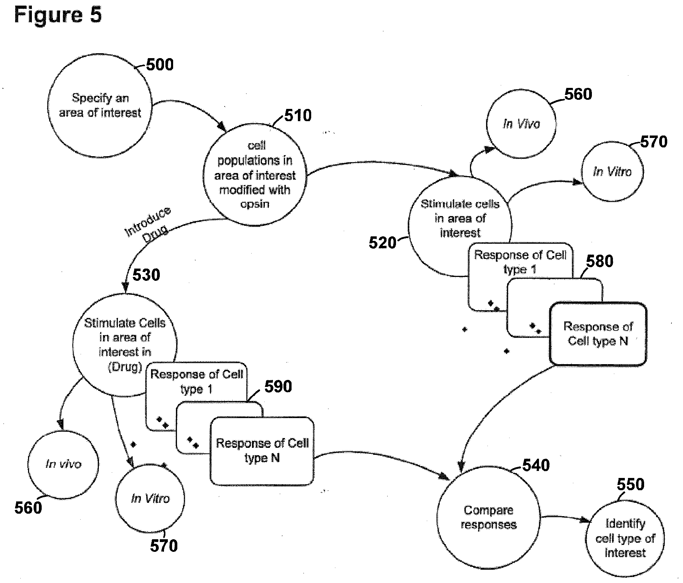

**FIG. 5** shows a model, in accordance with an example embodiment of the present disclosure.

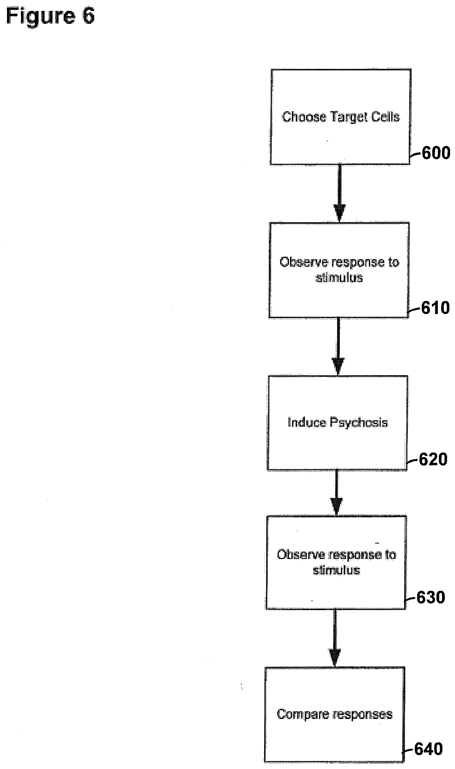

**FIG. 6** shows a method of identifying a cell of interest, in accordance with an example embodiment.

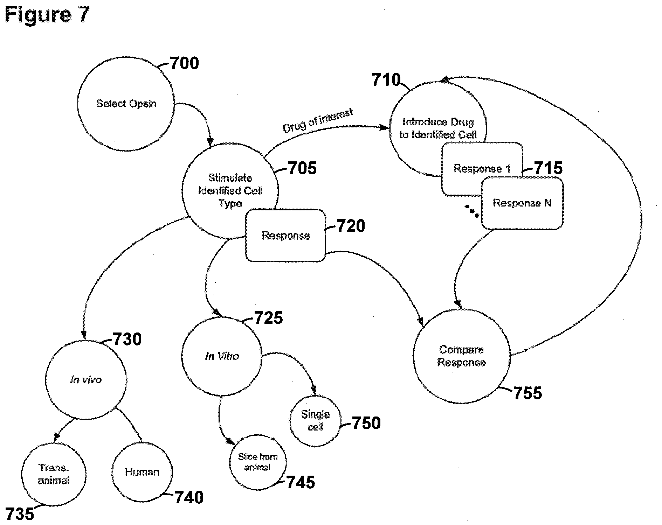

**FIG. 7** shows a model for characterizing neural disorders, in accordance with an example embodiment of the present disclosure.

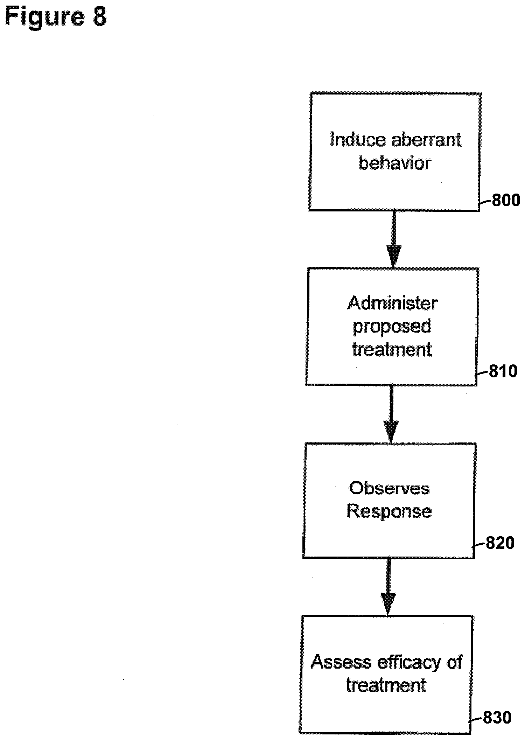

**FIG. 8** shows a method of determining the efficacy of a treatment, in accordance with an example embodiment.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the scope of the disclosure including aspects defined in the claims.

### DETAILED DESCRIPTION OF THE INVENTION

Described herein are non-human animals with psychosis induced by activating a light-responsive opsin expressed on the plasma membrane of a subject of layer V pyramidal neurons of the prefrontal cortex, wherein activation of the light-response opsin induces depolarization of the membrane. The prefrontal cortex tissue slices from the non-human animals are also provided. The disclosure also provides methods of inducing psychosis in non-human animals and methods for identifying or screening a compound that may be used for treating psychosis using the non-human animals and tissue slices described herein.

As used herein, an "animal" is a mammal. Mammals include, but are not limited to, humans, farm animals, sport animals, pets (e.g., dogs, and cats), primates, mice, rats, and other rodents.

As used herein, the singular form "a", "an", and "the" includes plural references unless indicated otherwise.

### General Techniques

The practice of the present invention will employ, unless otherwise indicated, conventional techniques of molecular biology, microbiology, cell biology, biochemistry, nucleic acid chemistry, immunology, physiology, urology, and the pathophysiology drug addiction and reward-related behaviors which are well known to those skilled in the art. Such techniques are explained fully in the literature, such as, Molecular Cloning: A Laboratory Manual, second edition (Sambrook et al., 1989) and Molecular Cloning: A Laboratory Manual, third edition (Sambrook and Russel, 2001), (jointly referred to herein as "Sambrook"); Current Protocols in Molecular Biology (F.M. Ausubel et al., eds., 1987, including supplements through 2001); PCR: The Polymerase Chain Reaction, (Mullis et al., eds., 1994); Harlow and Lane (1988) Antibodies, A Laboratory Manual, Cold Spring Harbor Publications, New York; Harlow and Lane (1999) Using Antibodies: A Laboratory Manual, Cold Spring Harbor Laboratory Press, Cold Spring Harbor, NY (jointly referred to herein as "Harlow and Lane"), Beaucage et al. eds., Current Protocols in Nucleic Acid Chemistry, John Wiley & Sons, Inc., New York, 2000), Handbook of Experimental Immunology, 4th edition (D. M. Weir & C. C. Blackwell, eds., Blackwell Science Inc., 1987); and Gene Transfer Vectors for Mammalian Cells (J. M. Miller & M. P. Calos, eds., 1987). Other useful references include Harrison's Principles of Internal Medicine (McGraw Hill; J. Isseleacher et al., eds.), and Addiction Research Methods, (Miller et al, eds., 2010; Wiley-Blackwell, United Kingdom).

### Light-responsive opsin proteins

Provided herein are optogenetic-based compositions and methods for selectively depolarizing a subject of layer V pyramidal neurons of the prefrontal cortex, wherein the depolarization of these neurons induces psychosis of the animal. In some embodiments, the schizophrenia is induced. Optogenetics refers to the combination of genetic and optical methods used to control specific events in targeted cells of living tissue, even within freely moving mammals and other animals, with the temporal precision (millisecond-timescale) needed to keep pace with functioning intact biological systems. Optogenetics requires the introduction of fast light-responsive channel or pump proteins to the plasma membranes of target neuronal cells that allow temporally precise manipulation of neuronal membrane potential while maintaining cell-type resolution through the use of specific targeting mechanisms.

Light-responsive opsins that may be used in the present invention include opsins that induce depolarization of the cell membrane of neurons by light. Examples of such opsins are shown in Table 1 below.

**Table 1 shows identified opsins for excitation and modulation across the visible spectrum:**

| **Opsin Type** | **Biological Origin** | **Wavelength Sensitivity** | **Defined action** |
|---|---|---|---|
| ***VChR1*** | *Volvox carteri* | 589nm utility | Excitation (depolarization) |
| | | 535nm max | |
| *DChR* | *Dunaliella salina* | 500nm max | Excitation (depolarization) |
| *ChR2* | *Chlamydomonas reinhardtii* | 470nm max | Excitation (depolarization) |
| | | 380-405nm utility | |
| *ChETA* | *Chlamydomonas reinhardtii* | 470nm max | Excitation (depolarization) |
| | | 380-405nm utility | |
| *SFO* | *Chlamydomonas reinhardtii* | 470nm max | Excitation (depolarization) |
| | | 530nm max | Inactivation |
| *SSFO* | *Chlamydomonas reinhardtii* | 445nm max | Step-like activation (depolarization) |
| | | 590nm; 390-400nm | Inactivation |
| *C1V1* | *Volvox carteri Chlamydomonas reinhardtii* | 542nm max | Excitation (depolarization) |
| *C1V1 E122* | *Volvox carteri and Chlamydomonas reinhardtii* | 546nm max | Excitation (depolarization) |
| *C1V1 E162* | *Volvox carteri Chlamydomonas reinhardtii* | 542nm max | Excitation (depolarization) |
| *C1V1 E122*/*E162* | *Volvox carteri and Chlamydomonas reinhardtii* | 546nm max | Excitation (depolarization) |

As used herein, a light-responsive opsin (such as ChR2, VChR1, DChR, and ChETA) includes naturally occurring protein and functional variants, fragments, fusion proteins comprising the fragments or the full length protein. In some embodiments, the signal peptide may be removed. A variant may have an amino acid sequence at least about any of 90%, 91%, 92%, 93%, 94%, 95%, 96%, 97%, 98%, 99%, or 100% identical to the naturally occurring protein sequence. A functional variant may have the same or similar depolarization function as the naturally occurring protein.

### Enhanced intracellular transport amino acid motifs

The present disclosure provides for the modification of light-responsive opsin proteins expressed in a cell by the addition of one or more amino acid sequence motifs which enhance transport to the plasma membranes of mammalian cells. Light-responsive opsin proteins having components derived from evolutionarily simpler organisms may not be expressed or tolerated by mammalian cells or may exhibit impaired subcellular localization when expressed at high levels in mammalian cells. Consequently, in some embodiments, the light- responsive opsin proteins expressed in a cell can be fused to one or more amino acid sequence motifs selected from the group consisting of a signal peptide, an endoplasmic reticulum (ER) export signal, a membrane trafficking signal, and/or an N-terminal golgi export signal. The one or more amino acid sequence motifs which enhance light- responsive opsin protein transport to the plasma membranes of mammalian cells can be fused to the N-terminus, the C-terminus, or to both the N- and C-terminal ends of the light- responsive opsin protein. Optionally, the light- responsive opsin protein and the one or more amino acid sequence motifs may be separated by a linker. In some embodiments, the light-responsive opsin protein can be modified by the addition of a trafficking signal (ts) which enhances transport of the protein to the cell plasma membrane. In some embodiments, the trafficking signal can be derived from the amino acid sequence of the human inward rectifier potassium channel Kᵢᵣ2.1. In other embodiments, the trafficking signal can comprise the amino acid sequence KSRITSEGEYIPLDQIDINV.

Additional protein motifs which can enhance light- responsive opsin protein transport to the plasma membrane of a cell are described in U.S. Patent Application No. 12/041,628. In some embodiments, the signal peptide sequence in the protein can be deleted or substituted with a signal peptide sequence from a different protein.

### Light-responsive channel proteins

In some aspects, the light-responsive opsin protein is a light-responsive channel protein. In some aspects of the methods provided herein, one or more members of the Channelrhodopsin family of light-responsive ion channels are expressed on the plasma membranes of the subset of layer V pyramidal neurons in the prefrontal cortex.

In some aspects, the light-responsive cation channel protein can be derived from *Chlamydomonas reinhardtii,* wherein the cation channel protein can be capable of mediating a depolarizing current in the cell when the cell is illuminated with light. In another embodiment, the light-responsive cation channel protein can comprise an amino acid sequence at least about 90%, 91%, 92%, 93%, 94%, 95%, 96%, 97%, 98%, 99%, or 100% identical to the sequence shown in SEQ ID NO:1. The light used to activate the light-responsive cation channel protein derived from *Chlamydomonas reinhardtii* can have a wavelength between about 460 and about 495 nm or can have a wavelength of about 470 nm. Additionally, the light can have an intensity of at least about 100 Hz. In some embodiments, activation of the light-responsive cation channel derived from *Chlamydomonas reinhardtii* with light having an intensity of 100 Hz can cause depolarization-induced synaptic depletion of the neurons expressing the light-responsive cation channel. The light-responsive cation channel protein can additionally comprise substitutions, deletions, and/or insertions introduced into a native amino acid sequence to increase or decrease sensitivity to light, increase or decrease sensitivity to particular wavelengths of light, and/or increase or decrease the ability of the light-responsive cation channel protein to regulate the polarization state of the plasma membrane of the cell. Additionally, the light-responsive cation channel protein can contain one or more conservative amino acid substitutions and/or one or more non-conservative amino acid substitutions. The light-responsive cation channel protein comprising substitutions, deletions, and/or insertions introduced into the native amino acid sequence suitably retains the ability to depolarize the plasma membrane of a neuronal cell in response to light.

In other embodiments, the light-responsive cation channel protein can be a step function opsin (SFO) protein or a stabilized step function opsin (SSFO) protein that can have specific amino acid substitutions at key positions throughout the retinal binding pocket of the protein. In some embodiments, the SFO protein can have a mutation at amino acid residue C128 of SEQ ID NO:1. In other embodiments, the SFO protein has a C128A mutation in SEQ ID NO:1. In other embodiments, the SFO protein has a C128S mutation in SEQ ID NO:1. In another embodiment, the SFO protein has a C128T mutation in SEQ ID NO:1. In some embodiments, the SSFO protein can have a mutation at amino acid residues C128 and D156 of SEQ ID NO:1. In other embodiments, the SSFO protein has a C128S mutation and a D156A mutation in SEQ ID NO:1. In some embodiments, the SFO protein can comprise an amino acid sequence at least about 90%, 91%, 92%, 93%, 94%, 95%, 96%, 97%, 98%, 99%, or 100% identical to the sequence shown in SEQ ID NO:2 or SEQ ID NO:3.

In other embodiments, the light-responsive cation channel protein can be a CIV1 chimeric protein derived from the VChR1 protein of *Volvox carteri* and the ChR1 protein from *Chlamydomonas reinhardti,* wherein the protein comprises the amino acid sequence of VChR1 having at least the first and second transmembrane helices replaced by the first and second transmembrane helices of ChR1; is responsive to light; and is capable of mediating a depolarizing current in the cell when the cell is illuminated with light. Additionally, in some embodiments, described herein are polypeptides comprising substituted or mutated amino acid sequences, wherein the mutant polypeptide retains the characteristic light-responsive nature of the precursor C1V1 chimeric polypeptide but may also possess altered properties in some specific aspects. For example, the mutant light-responsive C1V1 chimeric proteins described herein can exhibit an increased level of expression both within an animal cell or on the animal cell plasma membrane; an altered responsiveness when exposed to different wavelengths of light, particularly red light; and/or a combination of traits whereby the chimeric C1V1 polypeptide possess the properties of low desensitization, fast deactivation, low violet-light activation for minimal cross-activation with other light-responsive cation channels, and/or strong expression in animal cells. In some embodiments, the C1V1 protein can comprise an amino acid sequence at least about 90%, 91%, 92%, 93%, 94%, 95%, 96%, 97%, 98%, 99%, or 100% identical to the sequence shown in SEQ ID NOs:4, 5, 6, or 7.

Further disclosure related to light-responsive cation channel proteins can be found in U.S. Patent Application Publication No. 2007/0054319 and International Patent Application Publication Nos. WO 2009/131837 and WO 2007/024391. Further disclosure related to SFO or SSFO proteins can be found in International Patent Application Publication No. WO 2010/056970 and U.S. Provisional Patent Application Nos. 61/410,704 and 61/511,905. Further disclosure related to C1V1 chimeric cation channels as well as mutant variants of the same can be found in U.S. Provisional Patent Application Nos. 61/410,736, 61/410,744, and 61/511,912.

### Polynucleotides encoding light-responsive opsin proteins

The disclosure also provides polynucleotides comprising a nucleotide sequence encoding a light-responsive opsin protein described herein. In some embodiments, the polynucleotide comprises an expression cassette. In some embodiments, the polynucleotide is a vector comprising the above-described nucleic acid. In some embodiments, the nucleic acid encoding a light-responsive opsin protein of the disclosure is operably linked to a promoter. Promoters are well known in the art. Any promoter that functions in a cholinergic interneuron can be used for expression of the light-responsive proteins and/or any variant thereof of the present disclosure. Initiation control regions or promoters, which are useful to drive expression of the light-responsive opsin proteins or variant thereof in a specific animal cell are numerous and familiar to those skilled in the art. Virtually any promoter capable of driving these nucleic acids can be used. In some embodiments, the promoter used to drive expression of the light-responsive protein can be the thymus cell antigen 1 (Thy1) promoter, which is capable of driving robust expression of transgenes in pyramidal neurons of the prefrontal cortex (*See, e.g.,* Arenkiel et al., Neuron 54, 205 (Apr 19, 2007)).

Also provided herein are vectors comprising a nucleotide sequence encoding a light-responsive opsin protein or any variant thereof described herein. The vectors that can be administered according to the present disclosure also include vectors comprising a nucleotide sequence which encodes an RNA (*e.g.,* an mRNA) that when transcribed from the polynucleotides of the vector will result in the accumulation of light-responsive proteins on the plasma membranes of target animal cells. Vectors which may be used, include, without limitation, lentiviral, HSV, adenoviral, and adeno-associated viral (AAV) vectors. Lentiviruses include, but are not limited to HIV-1, HIV-2, SIV, FIV and EIAV. Lentiviruses may be pseudotyped with the envelope proteins of other viruses, including, but not limited to VSV, rabies, Mo-MLV, baculovirus and Ebola. Such vectors may be prepared using standard methods in the art.

In some embodiments, the vector is a recombinant AAV vector. AAV vectors are DNA viruses of relatively small size that can integrate, in a stable and site-specific manner, into the genome of the cells that they infect. They are able to infect a wide spectrum of cells without inducing any effects on cellular growth, morphology or differentiation, and they do not appear to be involved in human pathologies. The AAV genome has been cloned, sequenced and characterized. It encompasses approximately 4700 bases and contains an inverted terminal repeat (ITR) region of approximately 145 bases at each end, which serves as an origin of replication for the virus. The remainder of the genome is divided into two essential regions that carry the encapsidation functions: the left-hand part of the genome, that contains the *rep* gene involved in viral replication and expression of the viral genes; and the right-hand part of the genome, that contains the *cap* gene encoding the capsid proteins of the virus.

AAV vectors may be prepared using standard methods in the art. Adeno-associated viruses of any serotype are suitable (see, *e.g.,* Blacklow, pp. 165-174 of "Parvoviruses and Human Disease" J. R. Pattison, ed. (1988); Rose, Comprehensive Virology 3:1, 1974; P. Tattersall "The Evolution of Parvovirus Taxonomy" In Parvoviruses (JR Kerr, SF Cotmore. ME Bloom, RM Linden, CR Parrish, Eds.) p5-14, Hudder Arnold, London, UK (2006); and DE Bowles, JE Rabinowitz, RJ Samulski "The Genus Dependovirus" (JR Kerr, SF Cotmore. ME Bloom, RM Linden, CR Parrish, Eds.) p15-23, Hudder Arnold, London, UK (2006)). Methods for purifying for vectors may be found in, for example, U.S. Pat. Nos. 6,566,118, 6,989,264, and 6,995,006 and WO/1999/011764 titled "Methods for Generating High Titer Helper-free Preparation of Recombinant AAV Vectors". Preparation of hybrid vectors is described in, for example, PCT Application No. PCT/US2005/027091. The use of vectors derived from the AAVs for transferring genes *in vitro* and *in vivo* has been described (*See e.g.,* International Patent Application Publication Nos.: 91/18088 and WO 93/09239; U.S. Patent Nos.: 4,797,368, 6,596,535, and 5,139,941; and European Patent No.: 0488528). These publications describe various AAV-derived constructs in which the *rep* and/or *cap* genes are deleted and replaced by a gene of interest, and the use of these constructs for transferring the gene of interest *in vitro* (into cultured cells) or *in vivo* (directly into an organism). The replication defective recombinant AAVs according to the disclosure can be prepared by co-transfecting a plasmid containing the nucleic acid sequence of interest flanked by two AAV inverted terminal repeat (ITR) regions, and a plasmid carrying the AAV encapsidation genes (*rep* and *cap* genes), into a cell line that is infected with a human helper virus (for example an adenovirus). The AAV recombinants that are produced are then purified by standard techniques.

In some embodiments, the vector(s) for use in the methods described herein are encapsidated into a virus particle (*e.g*. AAV virus particle including, but not limited to, AAV1, AAV2, AAV3, AAV4, AAV5,AAV6, AAV7, AAV8, AAV9, AAV10, AAV11, AAV12, AAV13, AAV14, AAV15, and AAV16). Accordingly, the disclosure includes a recombinant virus particle (recombinant because it contains a recombinant polynucleotide) comprising any of the vectors described herein. Methods of producing such particles are known in the art and are described in U.S. Patent No. 6,596,535.

### Delivery of light-responsive opsin proteins

In some aspects, polynucleotides encoding the light-responsive opsin proteins disclosed herein (for example, an AAV vector) can be delivered directly to the pyramidal neurons of the prefrontal cortex of an animal using a needle, catheter, or related device, using neurosurgical techniques known in the art, such as by stereotactic injection (*See, e.g.,* Stein et al., J. Virol, 73:34243429, 1999; Davidson et al., PNAS, 97:3428-3432, 2000; Davidson et al., Nat. Genet. 3:219-223, 1993; and Alisky & Davidson, Hum. Gene Ther. 11:2315-2329, 2000), or fluoroscopy.

In some aspects, a light-responsive opsin proteins can be expressed in the pyramidal neurons of the prefrontal cortex of a transgenic animal. For example, a transgenic mouse line can be employed using Cre-recombinase under control of the thymus cell antigen 1 (Thy1) promoter. A Cre-inducible adeno-associated virus (AAV) vector carrying the light-responsive opsin gene can then be stereotaxically injected into the prefrontal cortex.

In other aspects, any of the light-responsive opsin proteins can be expressed in the pyramidal neurons of the prefrontal cortex of a transgenic animal. For example, a transgenic mouse line can be employed using ChR2 under control of the thymus cell antigen 1 (Thy1) promoter. Transgenic mice can be generated using standard pronuclear injection techniques (*See, e.g.,* Arenkiel et al., Neuron 54, 205 (Apr 19, 2007)).

Other methods to deliver the light-responsive proteins to pyramidal neurons can also be used, such as, but not limited to, transfection with ionic lipids or polymers, electroporation, optical transfection, impalefection, or via gene gun.

In some aspects, the disclosure provides non-human animals generated by any methods described herein. In some embodiments, the non-human animals comprise a light-responsive opsin protein expressed on the cell membrane of a subset of layer V pyramidal neurons of the prefrontal cortex of the animal, wherein the opsin induces depolarization of the membrane by light, and wherein the illumination of the opsin by the light induces psychosis of the animal. In some aspects, the disclosure provides a prefrontal cortex tissue slice comprising a subset of layer V pyramidal neurons of the prefrontal cortex from a non-human animal, wherein a light-responsive opsin is expressed on the cell membrane of the subset of layer V pyramidal neurons, and activation of the opsin by light induces depolarization of the membrane.

### Light Sources

Any device that is capable of applying light having a wavelength to activate the light-responsive proteins expressed in a neuron may be used to depolarize the neuron. For example, a light-delivery device for activating a light-responsive opsin protein to affect the membrane voltage of one or more neurons may be used. A light-delivery device can be configured to provide optical stimulus to a target region of the brain. The light-delivery device may comprise a base, a cannula guide that is attached to the base, and one or more optical conduits attached to the base via the cannula guide. The base may comprise one or more light delivery ports that are positioned to deliver light from the optical conduits to targeted tissue regions, such as the pyramidal neurons in the prefrontal cortex. The optical conduits may be optical fibers, where the proximal end of the fiber is attached to an optical light source, and the distal end is in communication with the light delivery ports. The optical light source may be capable of providing continuous light and/or pulsed light, and may be programmable to provide light in pre-determined pulse sequences. The light delivery device may have any number of optical conduits as may be desirable, *e.g*., 1, 2, 3, 4, 5, 10, 15, 20, etc. The optical conduits may each carry light of the same or different wavelengths. The delivered light may have a wavelength between 450 nm and 600 nm, such as yellow or green or blue light. The light delivery device may have any number of light delivery ports as may be desirable, *e.g.,* 1, 2, 3, 4, 5, 10, 15, 20, *etc.* In some variations, there may be the same number of light delivery ports as optical conduits while in other variations, there may be different number of optical conduits and light delivery ports. For example, there may be a single optical conduit that conveys light to two or more light delivery ports. Alternatively or additionally, a single optical conduit may connect to a single light delivery port. The cannula guide may be configured to help secure and align the optical conduits with the light delivery ports. In some embodiments, the light delivery device is configured to deliver light to the subset of layer V pyramidal neurons of the prefrontal cortex to induce depolarization of the opsin proteins expressed on the cell membrane of the subset of layer V pyramidal neurons. Light delivery devices may also comprise one or more measurement electrodes that may be configured for measuring neural activity. For example, measurement electrodes may record changes in the membrane potential (e.g., action potentials) and/or current flow across a membrane of one or more neurons as the neurons respond to a stimulus. In some variations, the measurement electrodes may measure the electrical response of one or more neurons to optical stimulation. Measurement electrodes may be extracellular or intracellular electrodes.

In other aspects, the light delivery device can be an implantable light source that does not require physical tethering to an external power source. The implantable light source can comprise an inner body, the inner body having at least one means for generating light which is configured to a power source. In some embodiments, the power source can be an internal battery for powering the light-generating means. In another embodiment, the implantable light source can comprise an external antenna for receiving wirelessly transmitted electromagnetic energy from an external source for powering the light-generating means. The wirelessly transmitted electromagnetic energy can be a radio wave, a microwave, or any other electromagnetic energy source that can be transmitted from an external source to power the light-generating means of the implantable light source. In one embodiment, the light-generating means is controlled by an integrated circuit produced using semiconductor or other processes known in the art.

In some aspects, the light means can be a light emitting diode (LED). In some embodiments, the LED can generate blue and/or green light. In other embodiments, the LED can generate amber, yellow and/or blue light. In some embodiments, several micro LEDs are embedded into the inner body of the implantable light source. In other embodiments, the light-generating means is a solid state laser diode or any other means capable of generating light. The light generating means can generate light having an intensity sufficient to activate the light-responsive proteins expressed on the plasma membrane of the nerves in proximity to the light source (such as a light cuff). In some embodiments, the intensity of the light reaching the cholinergic interneurons of the NAc or striatum produced by the light-generating means has an intensity of any of about 0.05 mW/mm², 0.1 mW/mm², 0.2 mW/mm², 0.3 mW/mm², 0.4 mW/mm², 0.5 mW/mm², about 0.6 mW/mm², about 0.7 mW/mm², about 0.8 mW/mm², about 0.9 mW/mm², about 1.0 mW/mm², about 1.1 mW/mm², about 1.2 mW/mm², about 1.3 mW/mm², about 1.4 mW/mm², about 1.5 mW/mm², about 1.6 mW/mm², about 1.7 mW/mm², about 1.8 mW/mm², about 1.9 mW/mm², about 2.0 mW/mm², about 2.1 mW/mm², about 2.2 mW/mm², about 2.3 mW/mm², about 2.4 mW/mm², about 2.5 mW/mm², about 3 mW/mm², about 3.5 mW/mm², about 4 mW/mm², about 4.5 mW/mm², about 5 mW/mm², about 5.5 mW/mm², about 6 mW/mm², about 7 mW/mm², about 8 mW/mm², about 9 mW/mm², or about 10 mW/mm², inclusive, including values in between these numbers.

In some aspects, the light-generating means can be externally activated by an external controller. The external controller can comprise a power generator which can be mounted to a transmitting coil. In some embodiments of the external controller, a battery can be connected to the power generator, for providing power thereto. A switch can be connected to the power generator, allowing an individual to manually activate or deactivate the power generator. In some embodiments, upon activation of the switch, the power generator can provide power to the light-generating means on the light source through electromagnetic coupling between the transmitting coil on the external controller and the external antenna of the implantable light source. The transmitting coil can establish an electromagnetic coupling with the external antenna of the implantable light source when in proximity thereof, for supplying power to the light-generating means and for transmitting one or more control signals to the implantable light source. In some embodiments, the electromagnetic coupling between the transmitting coil of the external controller and the external antenna of the implantable light source can be radio-frequency magnetic inductance coupling. When radio-frequency magnetic inductance coupling is used, the operational frequency of the radio wave can be between about 1 and 20 MHz, inclusive, including any values in between these numbers (for example, about 1 MHz, about 2 MHz, about 3 MHz, about 4 MHz, about 5 MHz, about 6 MHz, about 7 MHz, about 8 MHz, about 9 MHz, about 10 MHz, about 11 MHz, about 12 MHz, about 13 MHz, about 14 MHz, about 15 MHz, about 16 MHz, about 17 MHz, about 18 MHz, about 19 MHz, or about 20 MHz). However, other coupling techniques may be used, such as an optical receiver, infrared, or a biomedical telemetry system (*See, e.g.,* Kiourti, "Biomedical Telemetry: Communication between Implanted Devices and the External World, Opticon1826, (8): Spring, 2010).

Examples of light stimulation devices, including light sources, can be found in International Patent Application Nos: PCT/US08/50628 and PCT/US09/49936 and in Llewellyn et al., 2010, Nat. Med., 16(10): 161-165.

### Methods of inducing psychosis and screening compounds that affect psychotic states

The disclosure provides methods for inducing psychosis in a non-human animal comprising: administering a polynucleotide encoding a light-responsive opsin protein to the non-human animal, wherein the light-responsive opsin protein is expressed on the cell membrane of a subset of layer V pyramidal neurons in the prefrontal cortex of the non-human animal, and the protein is responsive to light and is capable of inducing membrane depolarization of a subset of layer V pyramidal neurons when the subset of layer V pyramidal neurons are illuminated with the light, whereby activating the protein by the light induces psychosis in the non-human animal. In some embodiments, the schizophrenia is induced. In some embodiments, disruption of social exploration is induced.

The disclosure also provide methods for inducing depolarization of the membrane of a subset of layer V pyramidal neurons in a prefrontal cortex tissue slice comprising: activating a light-responsive opsin protein expressed on the cell membrane of the subset of layer V pyramidal neurons in the prefrontal cortex tissue slice, wherein the light-responsive opsin protein is capable of inducing membrane depolarization of the neurons by light.

In some aspects, the non-human animals and prefrontal cortex tissue slices described herein may be used to identify, screen or test the effectiveness of a compound that is useful for treating psychosis (e.g., schizophrenia). For example, the disclosure provides methods of screening a compound that may be useful for treating psychosis, comprising measuring psychotic state of a non-human animal before and after administering the compound to the prefrontal cortex of the animal, wherein the psychotic state is induced by light activation of a light-responsive opsin expressed on the cell membrane of a subject of layer V pyramidal neurons in the animal, and activation of the opsin induces depolarization of the membrane; wherein an improvement in one or more of psychotic state measurements after the administration of the compound indicates that the compound may be useful for treating psychosis. In some embodiments, the psychotic state measurement is a behavioral measurement (such as social exploration). In some embodiments, the psychotic state measurement is a cellular measurement (such as electrophysiology measurement of depolarization patterns exhibited by a subset of layer V pyramidal neurons). In another embodiment, the method further comprises a step of administering a D2 agonist (such as quinpriole) to the animal before administration of the compound. In some embodiments the compound can be a D2 antagonist such as, but not limited to, sulpiride and haloperidol. In some embodiments the compound can be an L-type Ca²⁺ channel antagonist such as, but not limited to, nifedipine.

The disclosure also provides methods of screening a compound that may be useful for treating psychosis, comprising: measuring a psychotic state of a prefrontal cortex tissue slice before and after incubating the tissue slice with the compound, wherein the prefrontal cortex tissue slice comprises a subject of layer V pyramidal neurons and a light-responsive opsin is expressed on the cell membrane of the subject of layer V pyramidal neurons, wherein the psychotic state is induced by the membrane depolarization of the neurons induced by activation of the light-responsive opsin; wherein an improvement in one or more of a psychotic state readouts after incubation with the compound indicates that the compound may be useful for treating psychosis. In some embodiments, the psychotic state measurement is a cellular measurement. In another embodiment, further comprising a step of administering a D2 agonist (such as quinpirole) with the prefrontal cortex tissue slice before incubation with the compound. In some embodiments the compound can be a D2 antagonist such as, but not limited to, sulpiride and haloperidol. In some embodiments the compound can be an L-type Ca²⁺ channel antagonist such as, but not limited to, nifedipine.

### Exemplary Embodiments

The present disclosure is believed to be useful for the identification of neural cell populations involved in various psychiatric disorders. Specific applications of the present invention facilitate assessing disease models relating to neural cell populations linked to various psychiatric disorders. As many aspects of the example embodiments disclosed herein relate to and significantly build on previous developments in this field, the following discussion summarizes such previous developments to provide a solid understanding of the foundation and underlying teachings from which implementation details and modifications might be drawn including those found in the Examples. It is in this context that the following discussion is provided. While the present invention is not necessarily limited to such applications, various aspects of the invention may be appreciated through a discussion of various examples using this context.

The embodiments and specific applications discussed herein (including the Examples) may be implemented in connection with one or more of the above-described aspects, embodiments and implementations, as well as with those shown in the figures and described below. Reference may be made to the following Examples. For further details on light-responsive molecules and/or opsins, including methodology, devices and substances, reference may also be made to the following background publication: U.S. Patent Publication No. 2010/0190229, entitled "System for Optical Stimulation of Target Cells" to Zhang *et al.;* U.S. Patent Publication No. 2010/0145418, also entitled "System for Optical Stimulation of Target Cells" to Zhang *et al.;* and U.S. Patent Publication No. 2007/0261127, entitled "System for Optical Stimulation of Target Cells" to Boyden *et al.* Consistent with these publications, numerous opsins can be used in mammalian cells *in vivo* and *in vitro* to provide optical stimulation and control of target cells. For example, when ChR2 is introduced into a cell, light activation of the ChR2 channelrhodopsin results in excitation and firing of the cell. In instances when NpHR is introduced into a cell, light activation of the NpHR opsin results in inhibition of the cell. These and other aspects of the disclosures of the above-referenced patent applications may be useful in implementing various aspects of the present disclosure.

In some aspects, provided herein are methods of identifying neural populations involved in psychiatric disorders comprising: providing optical stimulation to a target neuron population that expresses a light-responsive opsin, measuring a first electrical pattern of the target neuron population in response to the optical stimulation, introducing a drug, known to induce psychosis, to the target neuron population, providing optical stimulation to the target neuron population, measuring a subsequent electrical pattern of the target neuron population in response to the optical stimulation, and comparing the first electrical pattern and the subsequent electrical pattern. In a further embodiment, identifying a subset of neurons associated with psychosis. In some embodiments, the subset of neurons is a subset of layer V pyramidal neurons. In some embodiments the target neuron cell population is in a patient. In some embodiments the drug can be a D2 agonist such as, but not limited to, quinpirole. In some embodiments the drug can be an NMDA receptor inhibitor such as, but not limited to, phencyclidine.

Turning to FIG. 5, a model for identifying cells of interest is depicted, consistent with an embodiment of the present disclosure. An area of interest within the brain is specified (500). The area of interest can be chosen based on previously identified characteristics of a disease of interest or known functions of the area, for example. One or more cell types within the area of interest are modified with an opsin (510). Each of the different cell types can be modified with a different opsin that responds to a different wavelength of light. In certain alternative embodiments, the different cell types are modified with the same opsin, but in different samples or patients. The different cell types in the area of interest are stimulated (520) with visible light in a range that is determined based on the opsin used to modify the particular cell type being stimulated. The response of each cell type is observed (580). A drug known to induce a particular state in a patient when observed on the macro level (instead of the cellular level) is introduced to the area of interest (530). The response of each cell type in the presence of the drug is observed (590). The responses of each cell type in the presence of the drug known to induce the particular state is compared (540) to the response of the same cell type to stimulation when the cells are in a "normal" or "natural" state (that is, without the drug being present). Based on the comparison a determination can be made as to whether one or more of the cell types is involved in creating the state triggered by the drug (550). If one or more of the responses in the presence of the drug differs from the baseline responses, the cell type with the changing response can be investigated further to determine whether the cell type is involved in causing the particular state being induced.

In various alternative and complementary embodiments, an area of interest can be studied *in vivo*(560), *in vitro* (570) or both. In embodiments studying an area of interest *in vitro,* slices of the area of interest that maintain the neural circuitry intact can be used. Multiple sets of the slices are used, with a different cell type within the area of interest being modified in each set. Alternatively, a single cell of each cell type in the area of interest can be studied. The alternative *in vitro* method allows for the response of the cell in isolation, as well as its effect on the surrounding cells, to be observed. The use of multiple sets of slices or single cells allows for a single opsin type to be used to modify all of the different cell types without causing all of the cells to fire at once.

In embodiments studying an area of interest *in vivo,* multiple subjects can be used with a different cell type being modified in each subject. Alternatively, different excitatory opsins, responsive to different wavelengths of light, can be used to modify the different cell types in the area of interest.

In certain more specific embodiments, the area of interest is layer V of the prefrontal cortex. The pyramidal neurons of Layer V are modified. The prefrontal cortex is believed to be involved in psychotic states such as schizophrenia. A drug such as quinpirole or PCP is introduced to the layer V pyramidal neurons. In comparing the response of the cells prior to the introduction of the drug to the response after the introduction of the drug it has been experimentally found that a subset of the layer V pyramidal neurons has a different response. This subset of layer V pyramidal neurons can be used to study the effectiveness of various treatments for schizophrenia or other psychosis.

Turning to FIG. 6, a method of determining a cell type of interest is disclosed. A target cell type is chosen (600). The choice of target cell can be made based on previous experimental results. The target cells are stimulated, and the response to the stimulation is observed (610). In certain more specific embodiments, stimulation of the target cells is achieved by activating a light-responsive molecule that has been introduced into the target cells. Psychosis is induced (620). This can be achieved by introducing a drug known to induce psychosis in a subject. The target cells are again stimulated and the response is observed (630). A comparison is made between the response of the target cells before the induction of psychosis and after the induction of psychosis (640). The comparison can be used to determine whether the target cells are involved in causing psychosis in the subject based on whether or not the cells react differently in the presence of the drug known to induce psychosis.

In certain embodiments of the present disclosure, both a D2 agonist such as quinpirole and a psychotomimetic drug, such as phencyclidine ("PCP'), produce schizophrenia-like behaviors in humans and animals. While these and other drugs act via different neuron receptors, the drugs both induce an activity-dependent depolarization phenotype in a subset of layer V pyramidal neurons in the prefrontal cortex ("PFC"). The activity dependent depolarization in these neurons disrupts the flow of information through the neurons and is causally linked to negative symptom-type social behaviors.

Various aspects of the present disclosure are directed to characteristics of a subset of layer V pyramidal neurons in the prefrontal cortex. Characteristics of the subset include activity-dependent depolarization that elicits bistability and disrupts the flow of information through these neurons. The disruptive bistability depends on a Ca²⁺ channel subtype (The L-type channel). This channel has been implicated previously in schizophrenia. Further, while depolarization of the subset neurons creates a non-social phenotype, blockade of the L-type channels corrects psychotomimetic-induced social dysfunction.

Certain aspects of the present disclosure are directed to identifying possible cellular underpinnings of psychotic behavior and impaired cognition as observed in schizophrenia and other related conditions. Optogenetic tools are used to activate cells without introducing an outside electrical source. This allows for a response to activation, of a target cell population that has been infected with an opsin, to be observed without, for example, other cells in the area also being activated and altering the results. In certain embodiments, the response of a cell is observed in the cells "natural" state, that is, without the presence of an additional stimulus known to cause behavior changes on a macro level. This response is then compared to the response of the cell of interest after a stimulus has been introduced.

Certain aspects of the present disclosure are directed to searching for specific neurons linked to prefrontal dysfunction in schizophrenia or other psychosis. A particular area of the brain or type of neural cell can be chosen for further examination, such as layer V pyramidal neurons within the PFC. Layer V pyramidal neurons are of interest with respect to schizophrenia and other forms of psychosis because D2 receptors in the PFC are concentrated on layer V pyramidal neurons and D2 receptors are known to play a role in schizophrenia. Further, layer V pyramidal neurons are output neurons of the neocortex and are responsible for signals including corollary discharge.

Various aspects of the present disclosure are used to verify connections between psychiatric disorders and specific neurons. Neurons of interest are modified to express channelrhodopsin-2 (ChR2) *in vivo.* The ChR2 in the modified neurons is stimulated. Cognitive impairment and other negative symptoms resulting from the stimulation are quantified. In models using Thy1::Chr18 transgenic mice, ChR2 expression is localized to layer V pyramidial neurons in the PFC. Modest optical stimulation (for example, 470 nm, 0.4mW, 5msec at 10Hz) is sufficient to disrupt social exploration of the mice without affecting normal locomotion. Increasing the frequency of stimulation (for example, 470nm, 0.4mW, 2.5msec at 40Hz) almost completely abolishes social behavior and elicits a variety of catatonic like behaviors. Assessing such results leads to the conclusion that the cells expressing ChR2 contribute to psychotic like behaviors. In certain embodiments, a finding such as this is used as a foundation for circuit level investigation of the cells expressing the opsin.

Certain aspects of the present disclosure are directed to combining opsin activation with pharmacological manipulations of target cells of interest. The D2 agonist quinpirole elicits schizophrenia-like behaviors in animals. Quinpirole can be delivered *in vivo,* or can be applied to slices from the area of interest. When using slices, network activity evoked by light is studied. As discussed in the Examples, including FIG. 2A, application of quinpirole changes the responses of a subset of layer V pyramidal neurons. The change in cell response includes some light flashes that no longer evoke spikes, new spikes unrelated to light flashes, and plateau potentials. Periods of high-frequency spiking is followed by a prolonged depolarization that outlasts direct stimulation by tens or hundreds of milliseconds. In certain instances this prolonged depolarization elicits further spiking. In other instances the depolarization produces a plateau-like depolarization above the spike threshold.

The induction of abnormal response using a drug known to induce psychosis allows for the testing of the effectiveness of antipsychotic drugs, and well as confirmation of the mechanisms of known antipsychotics. For example, application of the antipsychotic drug haloperidol reverses the effect of the quinpirole on the target cells. Similarity, the specific D2 antagonist sulpiride also reverses the effects of quinpirole on the target cells when used in the right doses. See the Examples for more discussion of the effect of quinpirole, haloperidol and sulpiride on D2 receptors, spike rate and other characteristics of the target cell.

As discussed briefly above, a subset of layer V pyramidal neurons was observed to display activity-dependent depolarization when quinpirole is present. The subset of cells with this reaction can be identified by a prominent sag and rebound after depolarization in response to a hyperpolarizing current pulse when quinpirole is not present. Layer V pyramidal neurons without the prominent sag and rebound do not exhibit activity-dependent depolarization in the presence of quinpirole. The two subpopulations of pyramidal neurons do not differ in their input resistance or membrane time constancies. However cells that exhibit the activity-dependent depolarization tend to be slightly more depolarized at rest.

In certain more specific embodiments, all of the neurons from Thyl:ChR2 transgenic mice in which quinpirole elicits the activity dependent depolarization strongly express ChR2, while conversely, most neurons strongly expressing ChR2 exhibit the activity-dependent depolarization. Thus, the subpopulation of layer V pyramidal neurons exhibiting the activity-dependent depolarization was substantially similar to the population activated by ChR2 that resulted in social and other behavioral abnormalities in Thy1::ChR2 transgenic mice. In transgenic mice, the activity-dependent depolarization can be elicited by current injection alone and did not require optogenetic stimulation.

The cells exhibiting the activity-dependent depolarization are morphologically distinct. The subset of layer V pyramidal neurons possesses a single large apical dendrite that does not bifurcate until it reaches superficial layers 2/3. The cells also have many projections within layer V. In contrast, cells without the activity-dependent depolarization have more heterogeneous morphologies, including apical dendrites that branch in layers 4 or 5. See the Examples for a more in-depth discussion of the differences between the subsets of layer 5 pyramidal neurons.

In certain embodiments of the present disclosure, the electrophysiological patterns are studied on the single cell level. Activity-dependent depolarization appears to elicit a range of notable cellular behaviors, including a marked depolarization that blocks further spiking, prolonged bistability, an increase in spiking elicited by current injection, and an afterdepolarization that outlasts the period of direct stimulations and can elicit additional spikes. Bistability is typically associated with rhythmic activity in the beta/gamma band. In certain embodiments, bistability peaks in the power spectra between 20 and 40 Hz.

In certain embodiments, the net effect of quinpirole on neural excitability (e.g., increase in spiking vs. depolarization block and associated phenomena) depended on the dose and duration of quinpirole application, as well as the input strength. In particular, increased spiking can occur during early quinpirole application or in response to weak depolarizing input, which evolves into a prolonged depolarization or bistability after longer quinpirole application or in response to stronger depolarizing input. The activity-dependent depolarization can be present in control conditions (before applying quinpirole). It was found that this phenomenon can be elicited in some circumstances by unusually-elevated levels of D2 receptor activation in a slice.

Certain aspects of the present disclosure are directed to whole-cell voltage clamping. This allows for the identification of ion channel currents effecting the activity-dependent depolarization. In the identified subset of layer V pyramidal neurons (discussed above), voltage-dependent Ca²⁺ currents contribute to the activity-dependent depolarization. A Ca²⁺ influx via L-type channels during an action potential activates Ca²⁺-dependent K⁺ currents that play a major role in spike repolarization. Strong D2 activation also enhances Ca²⁺ influx via L-type channels. In addition, strong D2 activation is expected to enhance the spike AHP, and selectively suppress spiking at short interspike intervals. D2 blockage is expected to suppress recruitment of Ca²⁺-dependent K⁺ currents, resulting in compromised spike repolarization, wider spikes, greater Na⁺ channel inactivation, higher spike threshold, and reduced spiking at short interspike intervals.

In certain embodiments of the present disclosure, the effects of quinpirole on a target cell population are compared to the effects of another psychotomimemic, such as phencyclidine (PCP). PCP produces a syndrome closely resembling schizophrenia in humans, and has long been used to model schizophrenia in animals. The psychotomimetic effects of PCP have long been attributed to NMDA receptor blockade. Surprisingly, it has been found that at a dose similar to that which blocks prefrontal NMDA receptors, PCP produced an activity-dependent depolarization and afterdepolarization outlasting the period of direct stimulation that were virtually identical to those elicited by quinpirole. In contrast to quinpirole, the effects are not blocked by sulpiride, but are blocked by nifedipine. This suggests that while PCP does not activate D2 receptors, it does produce an activity-dependent depolarization via L-type Ca²⁺ channels. PCP produces a similar range of effects via the activity-dependent depolarization as did quinpirole, including an afterdepolarization outlasting the period of direct stimulation, marked depolarization that blocked spiking, bistability, and persistent firing outlasting the period of direct stimulation. The activity-dependent depolarization is limited to the subpopulation of layer V pyramidal neurons that exhibited a prominent sag and rebound afterdepolarization in response to hyperpolarizing current injection. Interestingly, PCP was in some cases found to elicit wide action potentials that were blocked by nifedipine and may represent dendritic Ca²⁺ spikes. During responses to trains of light pulses in Thy1::ChR2 cortical slices, PCP (like quinpirole) suppressed spiking at short interspike intervals, suggesting that the effects of PCP on L-type Ca²⁺ currents (like those of quinpirole) enhance the recruitment of Ca²⁺-dependent K⁺ currents during action potentials.

The structurally and functionally distinct psychotomimetics quinpirole and PCP converge upon a common causally-important Ca²⁺ channel-dependent aberrant neural state. Accordingly, PCP, although not previously linked to this kind of pathway, appears to exert part of its psychotomimetic action by recruiting L-type Ca²⁺ channels. In the presence of PCP, the L-type calcium channel antagonist nifedipine ameliorates social deficits induced by PCP in mice. The nifedipine also reduces the incidence of catatonic-like behaviors elicited by PCP alone. Appropriate doses of nifedpine reduces excessive levels of calcium channel activation, thereby preventing prolonged spiking or bistability, and restores normal prefrontal output and other behaviors mediated by the prefrontal cortex.

Applying the observed responses of the subset of layer V pyramidal neurons to symptoms observed in patients with schizophrenia and/or other forms of mental illness, the activity-dependent depolarization represents a single cellular phenomenon that could support both perseverative and tangential behavior in prefrontal networks. Accordingly, the neurons, along with the induction of activity-dependent depolarization can be used as a disease model to study new potential treatments for diseases such as schizophrenia.

The present disclosure is believed to be useful for targeting of specific neural cell populations involved in various psychiatric disorders, and more specifically to psychosis and/or symptoms of psychosis. Specific applications of the present invention facilitate assessing, treating and characterizing psychosis and/or symptoms of psychosis by targeting specific neural cell populations linked thereto. As many aspects of the example embodiments disclosed herein relate to and significantly build on previous developments in this field, the following discussion summarizes such previous developments to provide a solid understanding of the foundation and underlying teachings from which implementation details and modifications might be drawn including those found in the Examples. It is in this context that the following discussion is provided. While the present invention is not necessarily limited to such applications, various aspects of the invention may be appreciated through a discussion of various examples using this context.

Embodiments of the present disclosure are directed towards targeting cells that are a subset of the layer V pyramidal neurons. The targeting of these cells can be particularly useful for generating, studying, treating or otherwise characterizing psychotic states.

Turning to FIG. 7, a model for the control and characterization of neural disorders is depicted, consistent with an embodiment of the present disclosure. A cell type that has been identified as having involvement in a neural disorder of interest is chosen. Depending on the characteristics and the known function of the cell type, an opsin is selected (700) to infect the identified cell type. For example, in certain more specific embodiments, an excitatory opsin such as ChR2 is chosen to infect the identified cell type. ChR2 is chosen when excitation of the identified cell type is desired. The identified cell type can be modified to exhibit aberrant behavior using a second opsin, or by introducing a drug known to induce the aberrant behavior of the disease being modeled. The behavior can be exhibited on the macro level and can include the outwards signs of the disease being modeled. In other embodiments the behavior monitored is on the cellular level and can include the response of the cell to a stimulus.

In various embodiments, as depicted in FIG. 7, the identified cell type can be located, and stimulated (705), *in vivo* (730) or *in vitro* (725). The identified cell type can be stimulated as a single cell (750) or as part of a slice from an animal (745), for example. For *in vivo* (730) modeling, the identified cell type can be in a transgenic animal (735) or a human (740), for example.

After the selected opsin has been delivered to the cell type of interest, the identified cell type is stimulated (705) and the response is observed (720). The response of the identified cell type can be observed when the cell is in its "normal" state or when a diseased state has been induced, or both. After the initial observations, a drug of interest is chosen. The drug can be chosen based on the identified cell type, the disease being modeled, previous observations of other drugs, a combination of these factors, or other factors guiding the determination of possible drugs to treat a specific disease. An initial dose of the drug is introduced to the identified cell (710), and the cell's response to stimulus in the presence of the drug is observed (715). The response is then compared to the response of the cell when the drug is not present (755).

In other embodiments, the response of the cell in the presence of the drug of interest is compared to the response of the cell in the presence of a drug known to induce aberrant behavior. The drug of interest can also be introduced to the identified cell type in addition to the drug known to induce aberrant behavior.

In certain embodiments, the dose of the drug of interest is modified and the identified cell type is stimulated again, based on the assessment. The response to the modified dose can then be compared to the response in the absence of the drug of interest as well as the response to the previous dosage of the drug of interest. The responses can also be compared to other drugs or treatments for the disease being modeled. This allows for the determination of the optimal dosage and drug for a specific disease. Because the response observed may be a behavioral response or a cellular response, an effective dosage and/or drug can be determined from a set of possible drugs.

In certain more specific embodiments, an identified cell type is a subset of the layer V pyramidal neurons. A drug such as PCP or quinpirole is introduced to the identified cell type to induce aberrant behavior consistent with a disease state to be modeled. The indentified cell type is then stimulated using visible light that activates the opsin introduced into the cell, and the response to the stimulation is observed. A second drug, the drug of interest, is then introduced to the identified cell type. The response to stimulus in the presence of the drug of interest is observed, and compared to the response of the cell in the diseased state. This comparison can be use to determine the efficacy of the drug of interest. It can also be used to determine whether modification should be made to the dose or to other aspects of the administration of the drug.

Turning to FIG. 8, a method for determining the efficacy of a proposed treatment, consistent with an embodiment of the present disclosure is depicted. An aberrant behavior can occur on the cellular level when investigating a treatment *in vitro.* When investigating a treatment *in vitro,* the aberrant behavior can be at the cellular level and/or at the macro level, including observable behavior of the patient. The aberrant behavior can be induced (800) by administration of a drug known to cause the aberrant behavior to be treated. A proposed treatment is administered (810) either to the patient (*in vivo*) or to a cell (or cells) (*in vitro*)*.* The response of the cell or patient to stimulus in the presence of the treatment is observed (820). The observations are then used to assess the efficacy of the treatment (830).

In various embodiments consistent with FIG. 8, the treatment can be a drug, electrical stimulus, or a behavioral treatment, for example. The response can be to a light stimulus, for example. The light stimulus can excite or inhibit the cells based on a chosen light-responsive molecule introduced to a cell population of interest.

Various embodiments described above and shown in the figures may be implemented together and/or in other manners. One or more of the items depicted in the drawings/figures can also be implemented in a more separated or integrated manner, or removed and/or rendered as inoperable in certain cases, as is useful in accordance with particular applications. For example, embodiments involving the disease modeling as discussed above may be implemented using a variety of different opsins and stimuli. Further, the modeling can occur *in vivo* or *in vitro.* In view of the description herein, those skilled in the art will recognize that many changes may be made thereto.

### EXAMPLES

Here we show that the D2 agonist quinpirole and the psychotomimetic phencyclidine, which produce schizophrenia-like behaviors in humans and animals but act via different receptors, converge upon a novel activity-dependent depolarization phenotype in a subset of layer V pyramidal neurons in the PFC. We show that this activity-dependent depolarization elicits bistability and disrupts the flow of information through these neurons, and that this disruptive bistability depends on a Ca²⁺ channel subtype (the L-type channel) which has been implicated in schizophrenia. Finally, we show that the cellular endophenotype of these neurons is causally linked to negative symptom-type social behaviors; selectively depolarizing these neurons optogenetically creates a non-social phenotype, whereas blockade of the L-type channels corrects psychotomimetic-induced social dysfunction. Together these data define a novel cellular mechanism of prefrontal dysfunction recruited by mechanisms relevant to schizophrenia and related disorders.

### Example 1: Psychotic-like behaviors induced in mice by optical stimulation of infralimbic layer V pyramidal neurons expressing ChR2.

We chose to conduct our search in layer V pyramidal neurons within the PFC, for two reasons. First, D2 receptors in the PFC are concentrated on layer V pyramidal neurons (3, 4), and D2 receptors play a critical role in schizophrenia and other forms of psychosis (5) as all known antipsychotics block the D2 receptor, whereas drugs that increase dopamine levels (e.g. L-Dopa, stimulants), and dopamine receptor agonists can cause psychosis in normal individuals, or exacerbate symptoms in patients with schizophrenia. Indeed, binding to cortical D2 receptors may specifically explain the superior efficacy of certain antipsychotics, particularly for cognitive and negative symptoms of schizophrenia (6). Second, layer V pyramidal neurons are the key output neurons of the neocortex, responsible for signals including corollary discharge, which, when deficient, could contribute to failures of self-monitoring that produce psychotic symptoms such as auditory hallucinations (7).

To verify that these neurons contribute to psychotic behavior in mice, we stimulated channelrhodopsin-2 (ChR2) in layer V pyramidal neurons within the PFC **(****FIG. 1A-B**) using the well-established Thyl::ChR18 transgenic mice, in which neocortical expression of ChR2 is chiefly localized to layer V pyramidal neurons (8). To quantify cognitive impairment and negative symptoms characteristic of schizophrenia, we measured social exploration in these mice, and to assess positive-like symptoms, we measured disorganized or catatonic behavior including stereotyped movements and rigidity.

### Materials and Methods

Optical stimulation of infralimbic layer V pyramidal neurons expressing ChR2-EYFP in Thy1::ChR2 transgenic mice was achieved by unilateral optical fiber placement above the infralimbic prefrontal cortex **(FIG. 1A-B).** Low frequency and high frequency gamma-band optical stimulation of layer V neurons was achieved with 473 nm blue light (10 Hz, 5-ms pulse width) and 473 nm blue light (40 Hz, 5-ms pulse width), respectively. To quantify cognitive impairment and negative symptoms characteristic of schizophrenia, social exploration was measured in these mice and for assessment of positive-like symptoms, disorganized or catatonic behavior including stereotyped movements and rigidity were measured.

### Results

We found that relatively modest optical stimulation (470 nm, 0.4 mW, 5 msec @ 10 Hz) was sufficient to markedly disrupt social exploration without affecting normal locomotion **(FIG. 1C-E).** Specifically, low frequency optical stimulation decreased social exploration of a novel juvenile in 6 of the 6 Thy1::ChR2-EYFP animals tested (p = 0.03; light on/ light off epochs interleaved) **(FIG. 1C-D).** Open field data demonstrated there was no difference on the overall velocity (left) or track length (right) of Thy1::ChR2 animals upon low frequency optical stimulation, indicating that normal locomotion was not affected with modest stimulation **(FIG. IE).** Increasing the frequency of stimulation (470 nm, 0.4 mW, 2.5 msec @ 40 Hz) almost completely abolished social behavior and elicited a variety of catatonic like behaviors **(FIG. F-H).** Specifically, increasing the frequency of optical stimulation of layer V pyramidal neurons almost completely abolished social exploration of a novel juvenile in 6 of the 6 animals tested (p < 0.01; light on/ light off epochs interleaved) **(FIG. F-G).** In addition, the high frequency 40 Hz optical stimulation significantly increased time spent in a catatonic-like rigid posture in 3 of the 6 mice tested (left; p < 0.05), and a trend was observed toward increased time spent engaging in repetitive side-to-side head movements in 2 of the 6 mice tested (right; p = 0.13) **(****FIG. 1H****).** These results further validate the idea that aberrant activity in layer V pyramidal neurons in the PFC can contribute to schizophrenia-like behaviors (9), and provide a foundation for circuit-level investigation. Furthermore, these results demonstrate that optical stimulation of ChR2-expressing layer V pyramidal neurons in the Thy1::ChR2-EYFP mouse line allows this mouse to be used as an animal model for schizophrenia.

### Example 2: A D2 agonist elicits an activity-dependent depolarization mediated by L-type Ca2+ channels in a subset of layer V pyramidal neurons that is reversible by D2 antagonist treatment.

Optical stimulation of ChR2-expressing layer pyramidal neurons induces schizophrenic-like behavior in Thy1: ChR2-EYFP transgenic mice. We therefore proceeded to explore processes by which pharmacologic manipulations relevant to schizophrenia could affect these layer V pyramidal neurons in the PFC. As discussed above, D2 receptors play a key role in schizophrenia, and the D2 agonist quinpirole elicits schizophrenia-like behaviors in animals (10, 11). To explore processes by which pharmacologic manipulations relevant to schizophrenia could affect these layer V pyramidal neurons in the PFC, prefrontal slices from Thy1::ChR2 mice were used to study network activity evoked by light in the presence or absence of quinpirole followed by treatment with known D2 antagonists **(****FIG. 2** **and** **3****).**

### Materials and Methods

We used prefrontal slices from Thy1::ChR2 mice to study network activity evoked by light. Brain slices isolated from Thy1::ChR2 transgenic mice underwent *in vitro* light stimulation and a layer V pyramidal neuron was monitored by electrophysiological recordings for cellular responses to trains of light flashes (470 nm, 1 msec) in the absence of a D2 agonist (control) or presence a D2 agonist (20 µM quinpirole). The brain slice was subsequently washed to remove the quinpirole and treated with either 0.2-2 µM haloperidol or 5 µM sulpiride, both of which are known D2 antagonists. The spike rate of the layer V pyramidal neurons and the amount of information transmitted by the spike rate was quantified as well as the number of spikes as a function of interspike interval.

Layer V pyramidal neurons were subsequently treated to hyperpolarizing and/or depolarizing current pulses in the presence of 20 µM quinpirole alone or in combination with a D2 antagonist, haloperidol (2 µM) or sulpiride (5 µM), or with an L-type Ca2+ channel antagonist, nifedipine (10 µM). Electrophysiological recordings monitored the cellular responses of the layer V pyramidal neurons to *in vitro* light stimulation when under the pharmacological conditions or treatments.

### Results

As illustrated in **FIG. 2A****,** application of quinpirole (20 µM; purple trace, lower panel) produced a marked change in the responses of a substantial fraction of layer V pyramidal neurons, such that some light flashes which previously evoked spikes no longer did so (arrows), while new spikes, unrelated to light flashes ("+") and plateau potentials ("p") appeared. In a subset of layer V pyramidal cells, we observed that periods of high-frequency spiking were followed by a prolonged depolarization, outlasting direct stimulation by tens or hundreds of milliseconds (**FIG. 2B****,** middle panel, purple trace; observed in 16/26 layer V pyramidal neurons). This activity-dependent depolarization could elicit further spiking, or produce a plateau-like depolarization above the spike threshold **(****FIG. 2B****).** Washout of quinpirole and simultaneous application of the antipsychotic drug haloperidol quickly reversed this effect (**FIG. 2B****,** lower panel, green trace; reversal with 0.2-10 µM haloperidol was observed in 9/9 cells), as did simultaneous application of quinpirole and the specific D2 antagonist sulpiride (reversal with 5 µM sulpride; observed in 2/2 cells).

Interestingly, an inverted-U-shaped curve was observed for the effects of D2 receptor activation on the amount of information that the spike rate of these neurons transmitted about the rate of light flashes; the amount of information was highest in control conditions, intermediate when D2 receptors were activated by quinpirole (20 µM) and lowest when D2 receptors were blocked with haloperidol (0.2-2 µM) or sulpiride (5 µM) (**FIG. 2C****;** p < 0.05, control vs. quinpirole; p < 0.01, control vs. haloperidol / sulpiride; 2-way ANOVA). We identified a mechanism for this relationship, in that there was also a U-shaped curve for the effects of D2 receptor activation on spiking in response to ChR2 stimulation: neurons spiked most in control conditions, and least when D2 receptors were blocked with haloperidol (0.2-2 µM) or sulpiride (5 µM) (**FIG. 2D****;** p < 0.01, control vs. quinpirole; p < 0.001, control vs. haloperidol / sulpiride; 2-way ANOVA). The lower spike rates when D2 receptors were activated by quinpirole or blocked by haloperidol or sulpiride were specifically linked to loss of spikes at short interspike intervals (**FIG. 2E****;** n = 4 cells per group), and indeed the effects of D2 agonists and antagonists appeared to reflect effects on repetitive action potentials **(****FIG. 2F****).** D2 activation with quinpirole (purple trace) enhanced the spike afterhyperpolarization (AHP, arrows in **FIG. 2F**), consistent with rendering subthreshold subsequent input that would elicit a second spike in control conditions. By contrast, D2 blockade with sulpiride widened action potentials, consistent with preventing spiking at short interspike intervals via a mechanism such as Na⁺ channel inactivation.

We characterized the activity-dependent depolarization further. First, we sought to determine if it would be present in a specific subpopulation of layer V pyramidal neurons defined by any particular identifying electrophysiological properties, gene expression pattern, or morphology. We found that nearly every layer V pyramidal cell with a prominent sag and rebound afterdepolarization ("*" in **FIG. 3A****,** top panel) in response to a hyperpolarizing current pulse exhibited the activity-dependent depolarization after the application of 20 µM quinpirole (16/17 cells), whereas 0/9 layer V pyramidal neurons without these properties exhibited the activity-dependent depolarization after applying quinpirole. These two subpopulations of pyramidal neurons did not differ in their input resistance or membrane time constants, although cells that exhibited the activity-dependent depolarization were slightly more depolarized at rest. Interestingly, all of the neurons from the Thy1::ChR2 transgenic mice in which quinpirole elicited the activity dependent depolarization strongly expressed ChR2 (14/14 cells); conversely most neurons strongly expressing ChR2 exhibited the activity-dependent depolarization (14/18 cells). Thus, the subpopulation of layer V pyramidal neurons exhibiting the activity-dependent depolarization was substantially similar to the population activated by ChR2 that resulted in social and other behavioral abnormalities in Thy1::ChR2 transgenic mice **(****FIG. 1****).** Importantly, the activity dependent depolarization could be elicited by current injection alone ("1" in **FIG. 3A****,** middle panel, purple trace) and did not require optogenetic stimulation nor the sort of network activity shown in **FIG. 2** to be manifested, as described below. We also identified morphological characteristics linked to the presence of the activity-dependent depolarization which may relate to previously-described morphological sub-networks in layer V of medial PFC (12): cells with the activity-dependent depolarization invariably possessed a single large apical dendrite that did not bifurcate until it reached superficial layers 2/3, along with many processes projecting within layer V (**FIG. 3B****;** left panel; n = 5), while in contrast, cells without the activity-dependent depolarization had more heterogeneous morphologies, often including apical dendrites that branched in layers 4 or 5 (right panel).

To develop an understanding of the cellular consequences of this effect, we explored associated electrophysiological patterns identified at the single-cell level. The activity-dependent depolarization appeared to elicit a range of notable cellular behaviors including a marked depolarization that blocked further spiking ("2" in **FIG. 3A**)**,** prolonged bistability ("3" in **FIG. 3A**), an increase in spiking elicited by current injection ("4" in **FIG. 3C**), and an afterdepolarization that outlasted the period of direct stimulation and could elicit additional spikes ("5" in **FIG. 3C**). The afterdepolarization, which was most commonly observed, was quantified as shown in **FIG. 3D****,** and the broader range of effects elicited by quinpirole was summarized in **FIG. 3E** as fraction of layer V pyramidal neurons with a prominent sag and rebound afterdepolarization that exhibited an afterdepolarization (ADP) following depolarizing current injection, depolarization blockade of spiking (depol block), bistability, or persistent firing that outlasted the period of depolarizing current injection. Of note, bistability was typically associated with rhythmic activity in the beta/gamma band (**FIG. 3F****;** 4/6 cells with bistability had peaks in their power spectra between 20 and 40 Hz).

We next sought to confirm that the activity dependent depolarization was mediated by D2 receptors. Indeed, we found that it could be blocked using either the antipsychotic haloperidol (0.2 - 10 µM; **FIG. 3A****,** lower panel, green trace; 9/9 cells), or the more specific D2 antagonist sulpiride (5 µM; **FIG. 3C****,** lower panel, green trace; 2/2 cells). The activity-dependent depolarization could also be elicited using lower doses of quinpirole; in 8/10 cells with a prominent sag during and rebound afterdepolarization following a hyperpolarizing current pulse, 5 µM (-) Quinpirole or 10 µM quinpirole elicited the activity-dependent depolarization and related phenomena (e.g. a prolonged afterdepolarization and/or bistability). We also found that the net effect of quinpirole on neural excitability (e.g. increase in spiking vs. depolarization block and associated phenomena) depended on the dose and duration of quinpirole application, as well as the strength of input. In particular, we often observed increased spiking during early quinpirole application or in response to weak depolarizing input, which evolved into a prolonged depolarization or bistability after longer quinpirole application or in response to stronger depolarizing input. The activity-dependent depolarization was in rare cases present in control conditions (before applying quinpirole), suggesting that this phenomenon could be elicited in some circumstances by unusually-elevated levels of D2 receptor activation in the slice; indeed, in such cases (n = 3), moderate doses of haloperidol (0.2 - 2 µM) could convert bistability into a more modest afterdepolarization, or block the activity dependent depolarization altogether.

We next sought to identify possible ion channel currents mediating the activity-dependent depolarization, in whole-cell voltage-clamp. After a series of 1 msec steps to 0 mV to simulate action potentials, quinpirole (20 µM) elicited a slowly activating depolarizing current at -30 mV, and increased a rapidly inactivating tail current at -60 mV; both of these effects were blocked by sulpiride (5 µM). These observations suggested that voltage-dependent Ca²⁺ currents contribute to the activity-dependent depolarization, and indeed application of voltage-dependent Ca²⁺ channel antagonists blocked the activity-dependent depolarization in 5/5 cells (5 mM Ni²⁺ or 100 µM nifedipine, n=2; 10 µM nifedipine, n=3; **FIG. 3G****,** bottom panel, gray trace). This role for L-type Ca²⁺ channels in the activity-dependent depolarization may inform the U-shaped curve for the effects of D2 receptor activation on spiking **(****FIG. 2C-F),** as Ca²⁺ influx via L-type channels during an action potential activates Ca²⁺-dependent K⁺ currents that play a major role in spike repolarization (13). Strong D2 activation would be expected to enhance Ca²⁺ influx via L-type channels (13), enhance the spike AHP, and selectively suppress spiking at short interspike intervals; conversely, D2 blockade would be expected to suppress recruitment of Ca²⁺-dependent K⁺ currents, resulting in compromised spike repolarization, wider spikes, greater Na⁺ channel inactivation, higher spike threshold, and ultimately, reduced spiking at short interspike intervals.

### Example 3: Phencyclidine (PCP) also elicits an activity-dependent depolarization via L-type Ca²⁺ channels that is reversible by treatment.

Having studied the effects of quinpirole on neurons that mediate prefrontal output, and identified a mechanism that disrupts their ability to transmit information, we next compared the effects of quinpirole to those of another psychotomimetic, phencyclidine (PCP). PCP produces a syndrome closely resembling schizophrenia in humans (14), and has long been used to model schizophrenia in animals.

### Materials and Methods

Brain slices isolated from Thy1::ChR2 transgenic mice underwent direct current stimulation and a layer V pyramidal neuron was monitored by electrophysiological recordings for cellular responses to depolarizing current pulses in the absence or presence of 5 µM PCP. The brain slice was subsequently treated with either 5 µM sulpiride or 10µM nifedipine and electrophysiological recording monitored the cellular response to treatment. To quantify cognitive impairment and negative symptoms characteristic of schizophrenia, social exploration was measured in mice treated with 4-15 mg/kg nifedipine alone or in combination with 5 mg/kg PCP.

Fraction of layer V pyramidal neurons with a prominent sag and rebound afterdepolarization that exhibited an afterdepolarization (ADP) following depolarizing current injection, depolarization blockade of spiking (depol block), bistability, or persistent firing that outlasted the period of depolarizing current injection, after application of 5 µM PCP(**FIG 4B****).** Top: Nifidepine impairs social exploration in a dose-dependent fashion (n = 8 mice in each group). Bottom: PCP impairs social exploration, but nifedipine ameliorates this deficit in PCP-treated mice in a dose-dependent fashion (n = 8 mice in each group) **(****FIG 4C****).** Responses of layer V pyramidal neurons to hyperpolarizing and depolarizing current pulses in a wild-type mouse, and in the TS2neo knock-in mouse designed as a CACNA1C gain of function gene(**FIG 4D****)** (20). * = p < 0.05, ** = p < 0.01. The effect was present in 4/4 mutant cells and 0/5 wild-type cells with a large sag and rebound depolarization in response to hyperpolarizing current injection (p < 0.01 by Fisher's exact test).

### Results

The psychotomimetic effects of PCP have long been attributed to NMDA receptor blockade; surprisingly, we found that at a dose similar to that which blocks prefrontal NMDA receptors (5 µM) (15), PCP produced an activity-dependent depolarization and afterdepolarization outlasting the period of direct stimulation that were virtually identical to those elicited by quinpirole (**FIG. 4A****;** observed in 6/12 layer V pyramidal neurons). These effects were not blocked by the D2 antagonist sulpiride (5 µM; n = 2 cells), but were blocked by the L-type Ca²⁺ channel antagonist nifedipine (10 µM; n = 2 cells), suggesting that while PCP does not activate D2 receptors, like quinpirole it produces an activity-dependent depolarization via L-type Ca²⁺ channels. Strikingly, PCP produced a similar range of effects via the activity-dependent depolarization as did quinpirole, including an afterdepolarization outlasting the period of direct stimulation, marked depolarization that blocked spiking, bistability, and persistent firing outlasting the period of direct stimulation **(****FIG. 4B****).** The activity-dependent depolarization was again limited to the subpopulation of layer V pyramidal neurons that exhibited a prominent sag and rebound afterdepolarization in response to hyperpolarizing current injection (the activity-dependent depolarization was observed in 6/9 of these neurons, and in 0/3 layer V pyramidal neurons without these properties). Interestingly, PCP was in some cases found to elicit wide action potentials that were blocked by nifedipine and may represent dendritic Ca²⁺ spikes **(****FIG. 4A****).** Finally, during responses to trains of light pulses in Thy1::ChR2 cortical slices, PCP (like quinpirole) suppressed spiking at short interspike intervals, suggesting that the effects of PCP on L-type Ca²⁺ currents (like those of quinpirole) enhance the recruitment of Ca²⁺-dependent K⁺ currents during action potentials.

If the structurally- and functionally-distinct psychotomimetics quinpirole and PCP do indeed converge upon a common causally-important Ca²⁺-channel-dependent aberrant neural state, a prediction emerges that PCP (not previously linked to this kind of pathway) could exert part of its psychotomimetic action by recruiting L-type Ca²⁺ channels. To test this novel hypothesis, we measured the effects of PCP (5 mg/kg) and the L-type calcium channel antagonist nifedipine (4, 15 mg/kg) on social behavior in mice. Consistent with the hypothesized U-shaped curve of effects, we found that nifedipine alone impaired sociability in a dose-dependent fashion (**FIG. 4C****,** top; p < 0.05, n = 8 mice per group), whereas in marked contrast, nifedipine ameliorated social deficits induced by PCP with a similar dose-dependence (**FIG. 4C****,** bottom; p < 0.05; n = 8 mice per group). Moreover, nifedipine (15 mg/kg; not shown) also reduced the incidence of catatonic-like behaviors, e.g. circling and rigidity, elicited by PCP alone (16). These data are consistent with the suggested hypothesis; nifedipine alone will reduce Ca²⁺ channel activation to levels that impair spike repolarization and repetitive spiking (thereby impairing prefrontal mediated-behaviors), but in combination with PCP, appropriate doses of nifedipine will reduce the excessive levels of calcium channel activation (preventing prolonged spiking or bistability), thereby restoring normal prefrontal output and rescuing behaviors mediated by prefrontal cortex.

### Discussion

Here we have identified and characterized a novel activity-dependent depolarization mediated by D2 receptor activation; this phenomenon is present in a defined subset of layer V pyramidal neurons in the PFC that when activated generate psychotomimetic-like behavior in mice. We also have determined that this activity-dependent depolarization generates bistability, aberrant spiking, and other cellular behaviors that disrupt the flow of information through these neurons, and thus holds face validity as a mechanism that could contribute to prefrontal dysfunction in schizophrenia and related forms of mental illness. We have found that surprisingly, a fundamentally distinct class of psychotomimetic (phencyclidine) also elicits a similar activity-dependent depolarization that was independent of D2 receptor activation. Finally, we determined that this shared activity-dependent depolarization depends on L-type Ca²⁺ channels, and that blocking L-type Ca²⁺ channels ameliorates the psychotomimetic effects of PCP in behaving mice.

These layer V neurons are well poised to affect the cognitive domains disrupted in schizophrenia. As shown by the effects of quinpirole, these neurons express D2 receptors, and prefrontal D2 receptors are involved in working memory and set-shifting tasks (9, 17); moreover, in the PFC, D2 receptors are located primarily on layer V pyramidal neurons (4), which send outputs from the PFC to other brain regions. Thus, via the effects shown in **FIG. 1A-D,** L-type Ca²⁺ channels in these layer V neurons could profoundly alter the information flowing out of the PFC to brain regions such as the striatum (18). We note that this hypothesized role for D2 receptors in gating prefrontal output could complement the stabilization of delay period activity by D1 receptors (19-23), and the gating of prefrontal input (24) by phasic dopamine release (25), which would preferentially activate D1 receptors (26). It is also intriguing to speculate that the range of effects mediated by the activity-dependent depolarization reported here could help to explain seemingly paradoxical clinical observations. Patients with schizophrenia or other forms of mental illness who are acutely psychotic are often highly tangential, with both speech and thinking shifting rapidly between topics that are connected only weakly, if at all. However the same patients, at almost the same time, can also be markedly perseverative, unable to shift their thinking or speech away from a single idea or word. The activity-dependent depolarization represents a single cellular phenomenon that could support both perseverative and tangential behavior in prefrontal networks. If D2 activation affects a subset of prefrontal neurons mediating output signals that trigger motor responses or corollary discharges (7, 9, 18, 27) via activity-dependent depolarization that enhances spiking in these neurons, the resulting aberrant or prolonged spiking **(****FIG. 2A****,B;** **FIG. 3C,E)** may give rise to promiscuous or inappropriate output signals corresponding to tangential behavior in processes mediated by cortical networks. Extremely high levels of D2 activation may in turn produce bistability **(****FIG. 3A****)** or depolarization blockade of spiking **(****FIG. 3A,E),** preventing output signaling and corresponding to perseverative or catatonic network behavior. Side effects of therapy may also be informed by this cellular phenotype, as D2 receptor blockade with high doses of antipsychotics will suppress prefrontal information transmission in this key population of output neurons **(****FIG. 2C****,D,F;** **FIG. 3A,C),** possibly contributing to psychomotor retardation and Parkinsonism, as observed clinically. The fact that excessive activation and blockade of D2 receptors can both produce similar phenotypes (decreased spiking), is reminiscent of other psychiatric phenomena, e.g. the U-shaped curve for the effects of DI receptor activation (19) and similar neurologic deterioration caused by over and underexpression of MeCP2 (28).

L-type Ca²⁺ channels have been implicated in schizophrenia by genome-wide association studies (29), although until now, there has been no clear hypothesis for their role. In fact, with few exceptions (30), it has been very difficult to link ion channels in specific neurons with symptoms of psychiatric disease. Genetic studies have also implicated L-type Ca²⁺ channels in bipolar disorder (31), which includes tangentiality and cognitive deficits similar to those seen in schizophrenia (32), and we have recently found that a mouse line designed to generate elevated L-type Ca²⁺ channel activity (33, 34) by prolonging L-type currents (35) elicited similar cellular dysfunction in layer V neurons **(****FIG. 4D****).** We also have found that L-type Ca²⁺ channel antagonism impaired social behavior, whereas the same dose rescued PCP-induced deficits in this behavior **(****FIG. 4C****).** Modest doses of nifedipine and other L-type calcium channel antagonists have occasionally shown promise for the treatment of schizophrenia (36-38) but conclusive studies are lacking. The findings reported here may inform the careful design and dose selection of such studies by providing a relevant cellular endophenotype that links a specific ion channel-driven phenomenon in a specific subpopulation of prefrontal neurons to symptomatology relevant to schizophrenia and other mental illnesses.

Although the foregoing invention has been described in some detail by way of illustration and example for purposes of clarity of understanding, the descriptions and examples should not be construed as limiting the scope of the invention.

### REFERENCES

1. P. S. Goldman-Rakic, L. D. Selemon, Schizophr Bull 23, 437 (1997).
2. D. M. Barch et al., Arch Gen Psychiatry 58, 280 (Mar, 2001).
3. M. S. Lidow, F. Wang, Y. Cao, P. S. Goldman-Rakic, Synapse 28, 10 (Jan, 1998).
4. N. Santana, G. Mengod, F. Artigas, Cereb Cortex 19, 849 (Apr, 2009).
5. A. Bonci, F. W. Hopf, Neuron 47, 335 (Aug 4, 2005).
6. R. M. Kessler et al., Neuropsychopharmacology 31, 1991 (Sep, 2006).
7. C. Frith, Lancet 346, 615 (Sep 2, 1995).
8. B. R. Arenkiel et al., Neuron 54, 205 (Apr 19, 2007).
9. M. Wang, S. Vijayraghavan, P. S. Goldman-Rakic, Science 303, 853 (Feb 6, 2004).
10. R. Y. Peng, R. S. Mansbach, D. L. Braff, M. A. Geyer, Neuropsychopharmacology 3, 211 (Jun, 1990).
11. A. F. Amsten, J. X. Cai, J. C. Steere, P. S. Goldman-Rakic, J Neurosci 15, 3429 (May, 1995).
12. Y. Wang et al., Nat Neurosci 9, 534 (Apr, 2006).
13. B. P. Bean, Nat Rev Neurosci 8, 451 (Jim, 2007).
14. D. C. Javitt, S. R. Zukin, Am J Psychiatry 148, 1301 (Oct, 1991).
15. R. Y. Wang, X. Liang, Neuropsychopharmacology 19, 74 (Jul, 1998).
16. G. T. Bolger, M. F. Rafferty, J. N. Crawley, S. M. Paul, P. Skolnick, Pharmacol Biochem Behav 25, 45 (Jul, 1986).
17. S. B. Floresco, O. Magyar, S. Ghods-Sharifi, C. Vexelman, M. T. Tse, Neuropsychopharmacology 31, 297 (Feb, 2006).
18. T. W. Robbins, Schizophr Bull 16, 391 (1990).
19. G. V. Williams, P. S. Goldman-Rakic, Nature 376, 572 (Aug 17, 1995).
20. D. Durstewitz, J. K. Seamans, T. J. Sejnowski, JNeurophysiol 83, 1733 (Mar, 2000).
21. K. Y. Tseng, P. O'Donnell, Cereb Cortex 15, 49 (Jan, 2005).
22. G. Winterer, D. R. Weinberger, Trends Neurosci 27, 683 (Nov, 2004).
23. K. Sidiropoulou et al., Nat Neurosci 12, 190 (Feb, 2009).
24. T. S. Braver, D. M. Batch, J. D. Cohen, Biol Psychiatry 46, 312 (Aug 1, 1999).
25. W. Schultz, Annu Rev Neurosci 30, 259 (2007).
26. Y. Goto, A. A. Grace, Nat Neurosci 8, 805 (Jim, 2005).
27. Y. Wang, P. S. Goldman-Rakic, Proc Natl Acad Sci USA 101, 5093 (Apr 6, 2004).
28. M. Chahrour, H. Y. Zoghbi, Neuron 56, 422 (Nov 8, 2007).
29. M. Nyegaard et al., Mol Psychiatry 15, 119 (Feb, 2010).
30. V. Krishnan et al., Cell 131, 391 (Oct 19, 2007).
31. P. Sklar et al., Mol Psychiatry 13, 558 (Jun, 2008).
32. M. F. Green, J Clin Psychiatry 67, e 12 (Oct, 2006).
33. I. Splawski et al., Cell 119, 19 (Oct 1, 2004).
34. S. R. Wersinger, Bett, G.C., Hess, R.A., Baizer, Rasmusson, R.L., paper presented at the Society for Neuroscience, 2008, Washington, DC, 2008.
35. C. F. Barrett, R. W. Tsien, Proc Natl Acad Sci USA 105, 2157 (Feb 12, 2008).
36. K. Yamada et al., Psychiatry Clin Neurosci 49, 237 (Aug, 1995).
37. K. Yamada et al., J Clin Psychopharmacol 16, 437 (Dec, 1996).
38. B. L. Schwartz, M. Fay-McCarthy, K. Kendrick, R. B. Rosse, S. I. Deutsch, Clin Neuropharmacol 20, 364 (Aug, 1997).

### SEQUENCES

The amino acid sequence of ChR2

MDYGGALSAVGRELLFVTNPVVVNGSVLVPEDQCYCAGWIESRGTNGAQTASNVLQWLAAGFSILLLMFYAYQT
WKSTCGWEEIYVCAIEMVKVILEFFFEFKNPSMLYLATGHRVQWLRYAEWLLTCPVILIHLSNLTGLSNDYSRR
TMGLLVSDIGTIVWGATSAMATGYVKVIFFCLGLCYGANTFFHAAKAYIEGYHTVPKGRCRQVVTGMAWLFFVS
WGMFPILFILGPEGFGVLSVYGSTVGHTIIDLMSKNCWGLLGHYLRVLIHEHILIHGDIRKTTKLNIGGTEIEV
ETLVEDEAEAGAVP  (SEQ ID NO:1)

The amino acid sequence of SFO:

MDYGGALSAVGRELLFVTNPVVVNGSVLVPEDQCYCAGWIESRGTNGAQTASNVLQWLAAGFSILLLMFYAYQT
WKSTCGWEEIYVCAIEMVKVILEFFFEFKNPSMLYLATGHRVQWLRYAEWLLTSPVILIHLSNLTGLSNDYSRR
TMGLLVSDIGTIVWGATSAMATGYVKVIFFCLGLCYGANTFFHAAKAYIEGYHTVPKGRCRQVVTGMAWLFFVS
WGMFPILFILGPEGFGVLSVYGSTVGHTIIDLMSKNCWGLLGHYLRVLIHEHILIHGDIRKTTKLNIGGTEIEV
ETLVEDEAEAGAVP (SEQ ID NO:2)

The amino acid sequence of SSFO:

MDYGGALSAVGRELLFVTNPVVVNGSVLVPEDQCYCAGWIESRGTNGAQTASNVLQWLAAGFSILLLMFYAYQT
WKSTCGWEEIYVCAIEMVKVILEFFFEFKNPSMLYLATGHRVQWLRYAEWLLTSPVILIHLSNLTGLSNDYSRR
TMGLLVSAIGTIVWGATSAMATGYVKVIFFCLGLCYGANTFFHAAKAYIEGYHTVPKGRCRQVVTGMAWLFFVS
WGMFPILFILGPEGFGVLSVYGSTVGHTIIDLMSKNCWGLLGHYLRVLIHEHILIHGDIRKTTKLNIGGTEIEV
ETLVEDEAEAGAVP    (SEQ ID NO:3)

The amino acid sequence of C1V1:

MSRRPWLLALALAVALAAGSAGASTGSDATVPVATQDGPDYVFHRAHERMLFQTSYTLENNGSVICIPNNGQC
FCLAWLKSNGTNAEKLAANILQWITFALSALCLMFYGYQTWKSTCGWEEIYVATIEMIKFIIEYFHEFDEPAV
IYSSNGNKTVWLRYAEWLLTCPVLLIHLSNLTGLKDDYSKRTMGLLVSDVGCIVWGATSAMCTGWTKILFFLI
SLSYGMYTYFHAAKVYIEAFHTVPKGICRELVRVMAWTFFVAWGMFPVLFLLGTEGFGHISPYGSAIGHSILD
LIAKNMWGVLGNYLRVKIHEHILLYGDIRKKQKITIAGQEMEVETLVAEEED     (SEQ ID NO:4)

The amino acid sequence of C1V1 (E122T):

MSRRPWLLALALAVALAAGSAGASTGSDATVPVATQDGPDYVFHRAHERMLFQTSYTLENNGSVICIPNNGQCF
CLAWLKSNGTNAEKLAANILQWITFALSALCLMFYGYQTWKSTCGWETIYVATIEMIKFIIEYFHEFDEPAVIY
SSNGNKTVWLRYAEWLLTCPVLLIHLSNLTGLKDDYSKRTMGLLVSDVGCIVWGATSAMCTGWTKILFFLISLS
YGMYTYFHAAKVYIEAFHTVPKGICRELVRVMAWTFFVAWGMFPVLFLLGTEGFGHISPYGSAIGHSILDLIAK
NMWGVLGNYLRVKIHEHILLYGDIRKKQKITIAGQEMEVETLVAEEED    (SEQ ID NO:5)

The amino acid sequence of C1V1 (E162T):

MSRRPWLLALALAVALAAGSAGASTGSDATVPVATQDGPDYVFHRAHERMLFQTSYTLENNGSVICIPNNGQC
FCLAWLKSNGTNAEKLAANILQWITFALSALCLMFYGYQTWKSTCGWEEIYVATIEMIKFIIEYFHEFDEPAV
IYSSNGNKTVWLRYATWLLTCPVLLIHLSNLTGLKDDYSKRTMGLLVSDVGCIVWGATSAMCTGWTKILFFLI
SLSYGMYTYFHAAKVYIEAFHTVPKGICRELVRVMAWTFFVAWGMFPVLFLLGTEGFGHISPYGSAIGHSILD
LIAKNMWGVLGNYLRVKIHEHILLYGDIRKKQKITIAGQEMEVETLVAEEED (SEQ ID NO:6)

The amino acid sequence of C1V1 (E122T/E162T):

MSRRPWLLALALAVALAAGSAGASTGSDATVPVATQDGPDYVFHRAHERMLFQTSYTLENNGSVICIPNNGQC
FCLAWLKSNGTNAEKLAANILQWITFALSALCLMFYGYQTWKSTCGWETIYVATIEMIKFIIEYFHEFDEPAV
IYSSNGNKTVWLRYATWLLTCPVLLIHLSNLTGLKDDYSKRTMGLLVSDVGCIVWGATSAMCTGWTKILFFLI
SLSYGMYTYFHAAKVYIEAFHTVPKGICRELVRVMAWTFFVAWGMFPVLFLLGTEGFGHISPYGSAIGHSILD
LIAKNMWGVLGNYLRVKIHEHILLYGDIRKKQKITIAGQEMEVETLVAEEED     (SEQ ID NO:7)

## Claims

1. A method of inducing psychosis in a non-human mammal comprising a light-responsive opsin expressed on the cell membrane of a subset of layer V pyramidal neurons in the prefrontal cortex, wherein the light-responsive opsin is *Chlamydomonas reinhardtii* ChR2 or a variant thereof having at least 90% amino acid sequence identity to SEQ ID NO:1, the method comprising activating the light-responsive opsin by light, wherein the light activation of the opsin induces depolarization of the cell membrane, thereby inducing a psychotic state in the mammal.

2. A method of screening a compound that may be useful for treating psychosis, the method comprising:
measuring a psychotic state of a non-human mammal before and after administering the compound to the prefrontal cortex of the mammal,
wherein the non-human mammal comprises a light-responsive opsin expressed on the cell membrane of a subset of layer V pyramidal neurons in the prefrontal cortex, wherein the light-responsive opsin is *Chlamydomonas reinhardtii* ChR2 or a variant thereof having at least 90% amino acid sequence identity to SEQ ID NO:1, and wherein the psychotic state is induced by light activation of the light-responsive opsin, wherein light activation of the opsin induces depolarization of the membrane;
wherein an improvement in one or more psychotic state measurements after the administration of the compound indicates that the compound may be useful for treating psychosis.

3. The method of claim 2, further comprising a step of administering a D2 agonist to the mammal before administration of the compound.

4. The method of claim 1 or 2, wherein the light-responsive opsin comprises an amino acid sequence at least 95% identical to the light-responsive cation channel protein of SEQ ID NO:1 derived from *Chlamydomonas reinhardtii* (ChR2).

5. The method of claim 1 or 2, wherein the light-responsive opsin is *Chlamydomonas reinhardtii* ChR2.

6. The method of claim 4 or 5, wherein expression of the light-responsive opsin is controlled by a Thy1 promoter.

7. The method of claim 2, wherein the psychotic state measurement is a behavioral measurement.

8. The method of claim 7, wherein the behavioral measurement is social exploration.

9. The method of claim 2, wherein the psychotic state measurement is a cellular measurement.

10. A method of screening a compound that may be useful for treating psychosis, comprising: measuring a cellular response in a prefrontal cortex tissue slice, obtained from a non-human mammal comprising a light-responsive opsin expressed on the cell membrane of a subset of layer V pyramidal neurons in the prefrontal cortex before and after incubating the tissue slice with the compound, wherein the light-responsive opsin is *Chlamydomonas reinhardtii* ChR2 or a variant thereof having at least 90% amino acid sequence identity to SEQ ID NO:1, wherein the cellular response is induced by the membrane depolarization of the neurons induced by activation of the light-responsive opsin; wherein an improvement in a cellular response readouts after incubation with the compound indicates that the compound may be useful for treating psychosis.

11. The method of claim 10, wherein the cellular response is activity-dependent depolarization.

## Patentansprüche

1. Verfahren zur Induktion einer Psychose bei einem nichtmenschlichen Säugetier, umfassend ein auf Licht ansprechendes Opsin, das auf der Zellmembran eines Subsatzes von Pyramidenneuronen der Schicht V im präfrontalen Kortex exprimiert wird, wobei das auf Licht ansprechende Opsin Chlamydomonas-reinhardtii-ChR2 oder eine Variante davon mit zumindest 90 % Aminosäuresequenzidentität mit Seq.-ID Nr. 1 ist, wobei das Verfahren das Aktivieren des auf Licht ansprechenden Opsins durch Licht umfasst, wobei die Lichtaktivierung des Opsins eine Depolarisation der Zellmembran induziert, wodurch ein psychotischer Zustand beim Säugetier induziert wird.

2. Verfahren zum Screenen einer Verbindung, die zur Behandlung einer Psychose nützlich sein könnte, wobei das Verfahren Folgendes umfasst:
Messen eines psychotischen Zustands eines nichtmenschlichen Säugetiers vor und nach der Verabreichung der Verbindung in den präfrontalen Kortex des Säugetiers,
wobei das nichtmenschliche Säugetier ein auf Licht ansprechendes Opsin umfasst, das auf der Zellmembran eines Subsatzes von Pyramidenneuronen der Schicht V im präfrontalen Kortex exprimiert wird, wobei das auf Licht ansprechende Opsin Chlamydomonas-reinhardtii-ChR2 oder eine Variante davon mit zumindest 90 % Aminosäuresequenzidentität mit Seq.-ID Nr. 1 ist und wobei der psychotische Zustand durch Lichtaktivierung des auf Licht ansprechenden Opsins induziert wird, wobei die Lichtaktivierung des Opsins eine Depolarisation der Membran induziert;
wobei eine Verbesserung einer oder mehrerer Messungen von psychotischen Zuständen nach der Verabreichung der Verbindung anzeigt, dass die Verbindung zur Behandlung einer Psychose nützlich sein könnte.

3. Verfahren nach Anspruch 2, das weiters einen Schritt des Verabreichens eines D2-Agonisten an das Säugetier vor der Verabreichung der Verbindung umfasst.

4. Verfahren nach Anspruch 1 oder 2, wobei das auf Licht ansprechende Opsin eine Aminosäuresequenz umfasst, die zu zumindest 95 % mit dem auf Licht ansprechenden Kationenkanalprotein der Seq.-ID Nr. 1 identisch ist, das von Chlamydomonas reinhardtii (ChR2) stammt.

5. Verfahren nach Anspruch 1 oder 2, wobei das auf Licht ansprechende Opsin Chlamydomonas-reinhardtii-ChR2 ist.

6. Verfahren nach Anspruch 4 oder 5, wobei die Expression des auf Licht ansprechenden Opsins durch einen Thy1-Promotor geregelt wird.

7. Verfahren nach Anspruch 2, wobei die Messung von psychotischen Zuständen eine Verhaltensmessung ist.

8. Verfahren nach Anspruch 7, wobei die Verhaltensmessung soziale Erkundung ist.

9. Verfahren nach Anspruch 2, wobei die Messung von psychotischen Zuständen eine zelluläre Messung ist.

10. Verfahren zum Screenen einer Verbindung, die zur Behandlung einer Psychose nützlich sein könnte, umfassend: Messen einer zellulären Antwort in einem Gewebeschnitt des präfrontalen Kortex, der von einem nichtmenschlichen Säugetier erhalten wurde und ein auf Licht ansprechendes Opsin umfasst, das auf der Zellmembran eines Subsatzes von Pyramidenneuronen der Schicht V im präfrontalen Kortex exprimiert wird, vor und nach einer Inkubation des Gewebeschnitts mit der Verbindung, wobei das auf Licht ansprechende Opsin Chlamydomonas-reinhardtii-ChR2 oder eine Variante davon mit zumindest 90 % Aminosäuresequenzidentität mit Seq.-ID Nr. 1 ist, wobei die zelluläre Antwort durch die Membrandepolarisation der Neuronen induziert wird, die durch Aktivierung des auf Licht ansprechenden Opsins induziert wird; wobei eine Verbesserung von Auslesungen einer zellulären Antwort nach der Inkubation mit der Verbindung anzeigt, dass die Verbindung zur Behandlung einer Psychose nützlich sein könnte.

11. Verfahren nach Anspruch 10, wobei die zelluläre Antwort aktivitätsabhängige Depolarisation ist.

## Revendications

1. Procédé d'induction d'une psychose chez un mammifère non humain comprenant une opsine sensible à la lumière exprimée sur la membrane cellulaire d'un sous-ensemble de neurones pyramidaux de la couche V dans le cortex préfrontal, où l'opsine sensible à la lumière est ChR2 de *Chlamydomonas reinhardtii* ou un variant de celle-ci présentant au moins 90 % d'identité de séquence d'acides aminés avec SEQ ID NO: 1, le procédé comprenant l'activation de l'opsine sensible à la lumière par la lumière, où l'activation de l'opsine par la lumière induit une dépolarisation de la membrane cellulaire, en induisant ainsi un état psychotique chez le mammifère.

2. Procédé de criblage d'un composé qui peut être utile pour traiter une psychose, le procédé comprenant :
la mesure d'un état psychotique d'un mammifère non humain avant et après l'administration du composé au cortex préfrontal du mammifère,
dans lequel le mammifère non humain comprend une opsine sensible à la lumière exprimée sur la membrane cellulaire d'un sous-ensemble de neurones pyramidaux de la couche V dans le cortex préfrontal, où l'opsine sensible à la lumière est ChR2 de *Chlamydomonas reinhardtii* ou un variant de celle-ci présentant au moins 90 % d'identité de séquence d'acides aminés avec SEQ ID NO: 1, et où l'état psychotique est induit par une activation de l'opsine sensible à la lumière par la lumière, où l'activation de l'opsine par la lumière induit une dépolarisation de la membrane ;
dans lequel une amélioration d'une ou de plusieurs mesures d'un état psychotique après l'administration du composé indique que le composé peut être utile pour traiter une psychose.

3. Procédé selon la revendication 2, comprenant en outre une étape consistant à administrer un agoniste D2 au mammifère avant l'administration du composé.

4. Procédé selon la revendication 1 ou 2, dans lequel l'opsine sensible à la lumière comprend une séquence d'acides aminés au moins 95 % identique à la protéine des canaux cationiques sensible à la lumière de SEQ ID NO: 1 dérivée de *Chlamydomonas reinhardtii* (ChR2).

5. Procédé selon la revendication 1 ou 2, dans lequel l'opsine sensible à la lumière est ChR2 de *Chlamydomonas reinhardtii.*

6. Procédé selon la revendication 4 ou 5, dans lequel l'expression de l'opsine sensible à la lumière est contrôlée par un promoteur Thy1.

7. Procédé selon la revendication 2, dans lequel la mesure d'un état psychotique est une mesure comportementale.

8. Procédé selon la revendication 7, dans lequel la mesure comportementale est une exploration sociale.

9. Procédé selon la revendication 2, dans lequel la mesure d'un état psychotique est une mesure cellulaire.

10. Procédé de criblage d'un composé qui peut être utile pour traiter une psychose, comprenant : la mesure d'une réponse cellulaire dans une coupe tissulaire de cortex préfrontal, obtenue à partir d'un mammifère non humain comprenant une opsine sensible à la lumière exprimée sur la membrane cellulaire d'un sous-ensemble de neurones pyramidaux de la couche V dans le cortex préfrontal avant et après une incubation de la coupe tissulaire avec le composé, où l'opsine sensible à la lumière est ChR2 de *Chlamydomonas reinhardtii* ou un variant de celle-ci présentant au moins 90 % d'identité de séquence d'acides aminés avec SEQ ID NO: 1, où la réponse cellulaire est induite par la dépolarisation de membrane des neurones induite par l'activation de l'opsine sensible à la lumière ; dans lequel une amélioration d'une mesure de la réponse cellulaire après l'incubation avec le composé indique que le composé peut être utile pour traiter une psychose.

11. Procédé selon la revendication 10, dans lequel la réponse cellulaire est une dépolarisation activité-dépendante.
